(19) Europäisches Patentamt
European Patent Office
Office européen des brevets

(11) Veröffentlichungsnummer: **0 243 645 B1**

(12) EUROPÄISCHE PATENTSCHRIFT

(45) Veröffentlichungstag der Patentschrift: **16.03.94**

(51) Int. Cl.[5]: **C07K 5/06**, A61K 37/02, A61K 37/64

(21) Anmeldenummer: **87103938.4**

(22) Anmeldetag: **18.03.87**

Die Akte enthält technische Angaben, die nach dem Eingang der Anmeldung eingereicht wurden und die nicht in dieser Patentschrift enthalten sind.

# (54) Verbindungen mit nootroper Wirkung, diese enthaltende Mittel und deren Verwendung bei der Behandlung und Prophylaxe cognitiver Dysfunktionen.

(30) Priorität: **27.03.86 DE 3610391**

(43) Veröffentlichungstag der Anmeldung:
**04.11.87 Patentblatt 87/45**

(45) Bekanntmachung des Hinweises auf die Patenterteilung:
**16.03.94 Patentblatt 94/11**

(84) Benannte Vertragsstaaten:
**AT BE CH DE ES FR GB GR IT LI LU NL SE**

(56) Entgegenhaltungen:
**EP-A- 0 050 800**
**EP-A- 0 116 276**

**BRITISH J. CLIN. PHARM., vol. 21, 1986; I. LICHTER et al., pp. 641-645***

**THE NEW ENGLAND JOURNAL OF MEDICINE, vol. 314, no. 26; S.H. CROOG et al., pp. 1657-1664***

(73) Patentinhaber: **HOECHST AKTIENGESELLSCHAFT**
**D-65926 Frankfurt(DE)**

(72) Erfinder: **Hock, Franz, Dr.**
**Altstadt 19**
**D-6110 Dieburg(DE)**
Erfinder: **Scholtholt, Josef, Prof. Dr.**
**Höhenstrasse 14**
**D-6450 Hanau 6(DE)**
Erfinder: **Urbach, Hansjörg, Dr.**
**Le Lavandoustrasse 41**
**D-6242 Kronberg/Taunus(DE)**
Erfinder: **Henning, Rainer, Dr.**
**Rotenhofstrasse 31**
**D-6234 Hattersheim am Main(DE)**
Erfinder: **Lerch, Ulrich, Dr.**
**Vorderwart 24**
**D-6238 Hofheim am Taunus(DE)**
Erfinder: **Nickel, Wolf-Ulrich, Dr.**
**Robert Stolz-Strasse 120**
**D-6232 Bad Soden am Taunus(DE)**
Erfinder: **Rüger, Wolfgang, Dr.**
**Parkstrasse 10**
**D-6233 Kelkheim (Taunus)(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft die Verwendung von Angiotensin-Converting-Enzyme-Inhibitoren (ACE-Inhibitoren) oder deren physiologisch verträglichen Salze als Arzneimittel mit nootroper (die cognitive Funktion verbessernder) Wirkung sowie deren Verwendung bei der Herstellung entsprechender pharmazeutischer Zubereitungen.

Aus EP-A 050 800 und EP-A 116 276 sind ACE-Inhibitoren mit unterschiedlichen heterocyclischen Strukturen bekannt, die zur Blutdrucksenkung eingesetzt werden können.

Für diese neuartige Verwendung kommen beispielsweise Verbindungen der Formel II in Betracht,

$$R^3OOC-\underset{R^4}{\overset{*}{CH}}-\underset{R^5}{N}-\underset{O}{\overset{}{C}}-\underset{R^1}{\overset{*}{CH}}-NH-\underset{COOR^2}{\overset{*}{CH}}-(CH_2)_n-R \qquad (II)$$

in welcher

n = 1 oder 2 ist,

R = Wasserstoff,
einen aliphatischen Rest mit 1 - 21 C-Atomen,
einen aromatischen Rest mit 6 - 12 C-Atomen,

$R^1$ Wasserstoff,
einen aliphatischen Rest mit 1 - 21 C-Atomen,
falls nicht von vorstehenden Definitionen schon umfaßt, die erforderlichenfalls geschützte Seitenkette einer natürlich vorkommenden $\alpha$-Aminosäure bedeutet,

$R^2$ und $R^3$ gleich oder verschieden sind und
Wasserstoff, einen aliphatischen Rest mit 1 - 21 C-Atomen einen alicyclischen Rest mit 3 - 20 C-Atomen,
einen aromatischen Rest mit 6 - 12 C-Atomen,
einen araliphatischen Rest mit 7-32 C-Atomen bedeuten und

$R^4$ und $R^5$ zusammen mit den sie tragenden Atomen ein mono-, bi- oder tricyclisches heterocyclisches Ringsystem mit 3 bis 15 Ring-C-Atomen bilden.

Unter einem aliphatischen Rest versteht man einen aliphatischen acyclischen Rest, d.h. einen Rest mit einer offenen, geraden oder verzweigten Kohlenstoffkette, wie beispielsweise Alkyl, Alkenyl, Alkinyl und entsprechende mehrfach ungesättigte Reste. Er ist vorzugsweise unsubstituiert oder, wie unten beispielsweise bei Carboxy, Carbamoyl, Aminoalkyl, Alkanoylaminoalkyl, Alkoxycarbonylaminoalkyl, Arylalkoxycarbonylaminoalkyl, Arylalkylaminoalkyl, Alkylaminoalkyl, Dialkylaminoalkyl, Alkylthioalkyl, Arylthioalkyl, Carboxyalkyl, Carbamoylalkyl, Alkoxycarbonylalkyl, Alkanoyloxyalkyl, Alkoxycarbonyloxyalkyl, Aroyloxyalkyl oder Aryloxycarbonyloxyalkyl beschrieben, monosubstituiert.

Ein alicyclischer Rest und der über eine offene Kohlenstoffkette verknüpfte entsprechende gegebenenfalls substituierte alicyclisch-aliphatische Rest ist ein vorzugsweise mono- bis pentacyclischer isocyclischer, nicht aromatischer Rest mit Einfach- oder unsymmetrisch verteilten Doppelbindungen, der auch verzweigt sein (d.h. offenkettige aliphatische Seitenketten tragen) kann, und der über ein Ring-C-Atom oder ein Seitenketten-C-Atom verknüpft ist. Er ist vorzugsweise unsubstituiert. Mehrere Ringe als Komponenten eines solchen Restes sind kondensiert, spiroverknüpft oder isoliert. Beispiele für solche Reste sind Cycloalkyl, Cycloalkenyl, Cycloalkylalkyl, Bicycloalkyl, Tricycloalkyl und von mono-, bi- oder oligocyclischen Terpenen abgeleitete Reste, wie Menthyl, Isomenthyl, Bornanyl, Bornyl, Caranyl, Epibornyl, Epiisobornyl, Isobornyl, Menthanyl, Neomenthyl, Neoisomenthyl, Pinanyl, Thujanyl; sie sind vorzugsweise unsubstituiert (aliphatische Seitenketten sind nach vorliegender Definition keine Substituenten).

Ein aromatischer Rest ist vorzugsweise Aryl wie Phenyl, Biphenylyl oder Naphthyl, das gegebenenfalls wie unten bei Aryl angegeben mono-, di- oder trisubstituiert ist. Von Aryl abgeleitete Reste, wie Aralkyl, Aryloxy, Arylthio oder Aroyl, vorzugsweise Benzoyl, können wie Aryl substituiert sein.

Unter einem araliphatischen Rest werden insbesondere Aralkylreste wie Arylalkyl, Diarylalkyl, Indanyl oder Fluorenyl verstanden, worin Aryl wie oben definiert ist und in der dort angegebenen Weise substituiert sein kann.

$R^4$ und $R^5$ kann mit den diese tragenden Atomen ein mono-, bi- oder tricyclisches heterocyclisches Ringsystem mit 3 bis 15 Ring-C-Atomen bilden, das im Ring vorzugsweise bis zu 2 S-Atome und bis zu 2

N-Atome, insbesondere bis zu 1 S-Atom aufweist.

Als solche Ringsgsteme kommen insbesondere jene aus der folgenden Gruppe in Betracht: Pyrrolidin (O); Tetrahydroisochinolin (A); Decahydroisochinolin (B); Octahydroindol (C); Indolin (Q); Octahydrocyclopenta-[b]pyrrol (D); 2-Azaspiro[4.5]decan (E); 2-Azaspiro[4.4]-nonan (F); Spiro[(bicyclo[2.2.1]-heptan)-2,3'-pyrrolidin] (G); Spiro[(bicyclo[2.2.2]octan)-2,3'-pyrrolidin] (H); 2-Azatricyclo[4.3.0.$1^{6,9}$]decan (I); Decahydrocyclohepta-[b]pyrrol (J); Octahydroisoindol (K); Octahydrocyclopenta-[c]pyrrol (L); 2,3,3a,4,5,7a-Hexahydroindol (M); 2-Aza-bicyclo[3.1.0]-hexan (N); 1,2,3,3a,4,6a-Hexahydrocyclopenta[b]pyrrol (P), die alle gegebenenfalls substituiert sein können. Pyrrolidin (O) und Thiazolidin (R) können z.B. durch ($C_6$-$C_{12}$)-Aryl (Phenyl, 2-Hydroxyphenyl, u.a.), ($C_6$-$C_{12}$)-Arylmercapto (wie Phenylmercapto) oder ($C_3$-$C_7$)-Cycloalkyl (wie Cyclohexyl) monosubstituiert sein. Tetrahydroisochinolin (A) kann z.B. im Arylteil bis zu 2 ($C_1$-$C_6$)-Alkoxyreste, vorzugsweise Methoxyreste tragen.

Entsprechendes gilt für die anderen Ringsysteme. Bevorzugt sind jedoch die unsubstituierten Systeme.

Bei Verbindungen der Formel II, die mehrere chirale Atome besitzen, kommen alle möglichen Diastereomere als Racemate oder Enantiomere, oder Gemische verschiedener Diastereomere in Betracht.

Die in Betracht kommenden heterocyclischen Ringsysteme weisen die folgenden Strukturformeln auf.

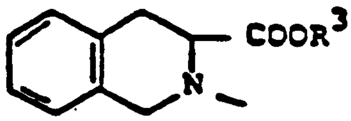

A

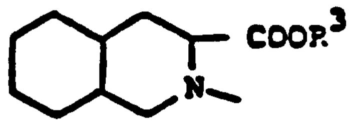

B

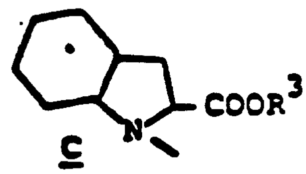

C

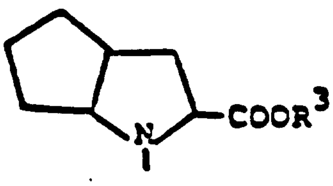

D

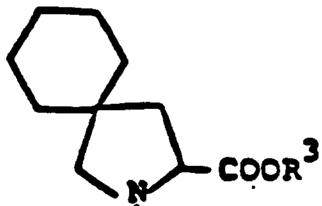

E

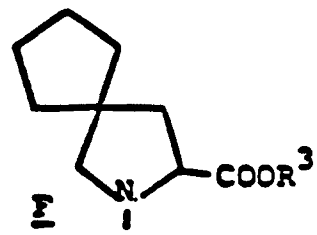

F

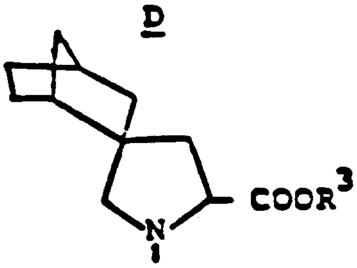

G

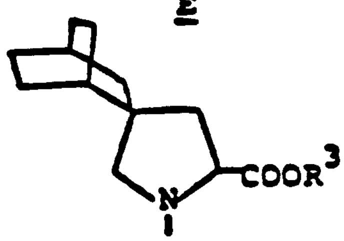

H

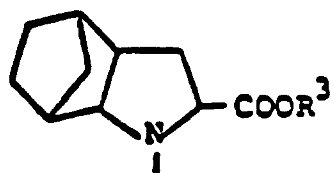

I

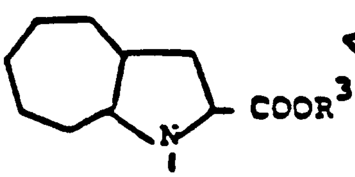

J

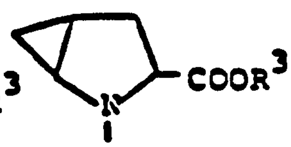

K

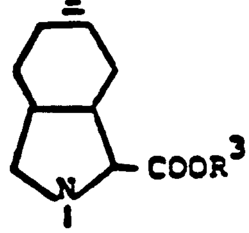

L

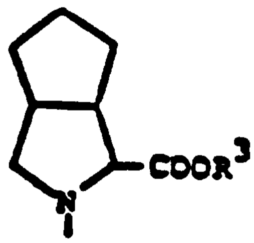

M

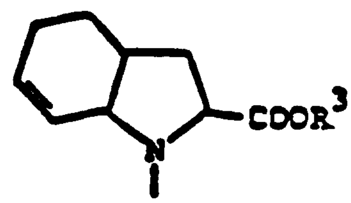

N

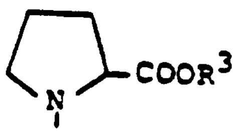

O

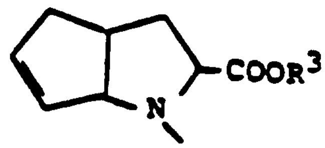

P

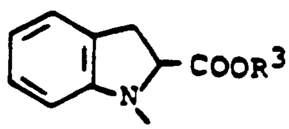

Q

Eine bevorzugte Ausführungsform ist dadurch gekennzeichnet, daß Verbindungen der Formel I, vorzugsweise solche der Formel II verwendet werden, in welchen

n = 1 oder 2 ist

R Wasserstoff,
Alkyl mit 1 - 8 C-Atomen,
Alkenyl mit 2 - 6 C-Atomen,
Aryl mit 6 - 12 C-Atomen,

$R^1$ Wasserstoff,
Alkyl mit 1 - 6 C-Atomen,
Alkenyl mit 2 - 6 C-Atomen,
Alkinyl mit 2 - 6 C-Atomen,

oder

die gegebenenfalls geschützte Seitenkette einer natürlich vorkommenden α-Aminosäure $R^1$-CH($NH_2$)-COOH bedeuten,

$R^2$ und $R^3$ gleich oder verschieden sind und Wasserstoff,
Alkyl mit 1 - 6 C-Atomen,
Alkenyl mit 2 - 6 C-Atomen,
Aryl mit 6 - 12 C-Atomen,
($C_6$-$C_{12}$)-Aryl-($C_1$-$C_4$)-alkyl,
($C_3$-$C_9$)-Cycloalkyl oder
bedeuten und

$R^4$ und $R^5$ die oben angegebene Bedeutung haben, insbesondere solche Verbindungen, in welchen

n = 1 oder 2 ist,

R ($C_1$-$C_6$)-Alkyl, ($C_2$-$C_6$)-Alkenyl, ($C_6$-$C_{12}$)-Aryl, insbesondere Methyl, Ethyl, oder Phenyl

$R^1$ Wasserstoff oder ($C_1$-$C_6$)Alkyl, ($C_2$-$C_6$)-Alkenyl, oder eine Seitenkette einer natürlich vorkommenden, gegebenenfalls geschützten α-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, ($C_1$-$C_6$)-Alkyl, ($C_2$-$C_6$)-Alkenyl oder ($C_6$-$C_{12}$)-Aryl-($C_1$-$C_4$)-alkyl, insbesondere aber Wasserstoff, ($C_1$-$C_4$) Alkyl oder Benzyl bedeuten und

$R^4$ und $R^5$ die oben angegebene Bedeutung haben.

Falls $R^1$ für eine Seitenkette einer geschützten natürlich vorkommenden α-Aminosäure steht, wie z.B. geschütztes Ser, Thr, Asp, Asn, Glu, Gln, Arg, Lys, Hyl, Cys, Orn, Cit, Tyr, Trp oder His, sind als Schutzgruppen in der Peptidchemie üblichen Gruppen bevorzugt (vgl. Houben-Weyl, Bd. XV/1 und XV/2). Im Falle, daß $R^1$ die geschützte Lysin-Seitenkette bedeutet, werden die bekannten Amino-Schutzgruppen, insbesondere aber Z, Boc oder ($C_1$-$C_6$)Alkanoyl bevorzugt. Als O-Schutzgruppen für Tyrosin kommen bevorzugt ($C_1$-$C_6$)-Alkyl, insbesondere Methyl oder Ethyl in Frage.

Besonders vorteilhaft können die folgenden Verbindungen erfindungsgemäß verwendet werden:
2-[N-(1-S-Carbethoxy-3-phenyl-propyl)-S-alanyl]-S-1,2,3,4-tetrahydroisochinolin-3-carbonsäure
2-[N-(1-S-Carbethoxy-3-cyclohexyl-propyl)-S-alanyl]-S-1,2, 3,4-tetrahydroisochinolin-3-carbonsäure
2-[N-(1-S-Carbethoxy-3-phenyl-propyl)-S-lysyl]-S-1,2,3,4-tetrahydroisochinolin-3-carbonsäure
2-[N-(1-S-Carbethoxy-3-phenyl-propyl)-O-ethyl-S-tyrosyl]-S-1,2,3,4-tetrahydroisochinolin-3-carbonsäure
2-[N-(1-S-Carbethoxy-3-phenyl-propyl)-S-alanyl]-(3S)-decahydroisochinolin-3-carbonsäure
1-[N-(1-S-Carbethoxy-3-phenyl-propyl)-S-alanyl]-(2S,3aS, 7aS)-octahydroindol-2-carbonsäure
1-[N-(1-S-Carbethoxy-3-cyclohexyl-propyl)-S-alanyl]-(2S,3aS, 7aS)-octahydroindol-2-carbonsäure
1-[N-(1-S-Carbethoxy-3-phenyl-propyl)-S-lysyl]-(2S,3aS,7aS)-octahydroindol-2-carbonsäure
1-[N-(1-S-Carbethoxy-3-cyclohexyl-propyl)-S-lysyl]-(2S,3aS, 7aS)-octahydroindol-2-carbonsäure
1-[N-(1-S-Carbethoxy-3-cyclohexyl-propyl)-S-lysyl]-(2S,3aS, 7aS)-octahydroindol-2-carbonsäure
1-[N-(1-S-Carbethoxy-3-phenyl-propyl)-O-methyl-S-tyrosyl]-(2S,3aS,7aS)-octahydroindol-2-carbonsäure
1-[N-(1-S-Carbethoxy-3-phenyl-propyl)-O-ethyl-S-tyrosyl]-(2S,3aS,7aS)-octahydroindol-2-carbonsäure
1-[N-(1-S-Carbethoxy-3-(3,4-dimethylphenyl-propyl)-S-alanyl]-(2S,3aS,7aS)-octahydroindol-2-carbonsäure
1-[N-[1-S-Carbethoxy-3-(4-fluorphenyl)-propyl]-S-alanyl]-(2S,3aS,7aS)-octahydroindol-2-carbonsäure
1-[N-[1-S-Carbethoxy-3-(4-methoxyphenyl)-propyl]-S-alanyl]-(2S,3aS,7aS)-octahydroindol-2-carbonsäure
1-[N-[1-S-Carbethoxy-3-(3,4-dimethoxyphenyl)-propyl]-S-alanyl]-(2S,3aS,7aS)-octahydroindol-2-carbonsäure
1-[N-(1-S-Carbethoxy-3-cyclopentylpropyl)-S-alanyl]-(2S,3aS, 7aS)-octahydroindol-2-carbonsäure
1-[N-(1-S-Carbethoxy-3-phenyl-propyl)-S-alanyl]-(2S,3aR, 7aS)-octahydroindol-2-carbonsäure
1-[N-(1-S-Carbethoxy-3-cyclohexyl-propyl)-S-alanyl]-(2S, 3aR,7aS)-octahydroindol-carbonsäure
1-[N-(1-S-Carbethoxy-3-phenyl-propyl)-S-lysyl]-(2S,3aR,7aS)-octahydroindol-2-carbonsäure
1-[N-(1-S-Carbethoxy-3-cyclohexyl-propyl)-S-lysyl]-(2S,3aR, 7aS)-octahydroindol-2-carbonsäure
1-[N-(1-S-Carbethoxy-3-phenyl-propyl)-O-ethyl-S-tyrosyl]-(2S,3aS,7aR)-octahydroindol-2-carbonsäure
1-[N-(1-S-Carbethoxy-3-phenyl-propyl)-S-alanyl]-(2S,3aR, 7aR)-octahydroindol-2-carbonsäure
1-[N-(1-S-Carbethoxy-3-phenyl-propyl)-S-lysyl]-(2S,3aR,7aS)-octahydroindol-2-carbonsäure
1-[N-(1-S-Carbethoxy-3-cyclohexyl-propyl)-S-alanyl]-(2S,3aR, 7aR)-octahydroindol-2-carbonsäure
1-[N-(1-S-Carbethoxy-3-cyclohexyl-propyl)-O-ethyl-S-tyrosyl]-(2S,3aR,7aR)-octahydroindol-2-carbonsäure
1-[N-(1-S-Carbethoxy-3-phenyl-propyl)-S-alanyl]-(2S,3aS, 7aR)-octahydroindol-2-carbonsäure
1-[N-(1-S-Carbethoxy-3-phenyl-propyl)-O-ethyl-S-tyrosyl]-(2S,3aS,7aS)-octahydroindol-2-carbonsäure
1-[N-(1-S-Carbethoxy-3,4-dimethylphenyl-propyl)-S-alanyl]-(2S,3aS,7aS)-octahydroindol-2-carbonsäure
1-[N-[1-S-Carbethoxy-3-(4-fluorphenyl)-propyl]-S-alanyl]-(2S,3aS,7aS)-octahydroindol-2-carbonsäure
1-[N-[1-S-Carbethoxy-3-(4-methoxyphenyl)-propyl]-S-alanyl]-(2S,3aS,7aS)-octahydroindol-2-carbonsäure
1-[N-[1-S-Carbethoxy-3-(3,4-dimethoxyphenyl)-propyl]-S-alanyl]-(2S,3aS,7aS)-octahydroindol-2-carbonsäure
1-[N-(1-S-Carbethoxy-3-cyclopentylpropyl)-S-alanyl]-(2S,3aS, 7aS)-octahydroindol-2-carbonsäure
2-[N-(1-S-Carbethoxy-3-phenyl-propyl)-S-alanyl]-cis-endo-2-azabicyclo[3.3.0]octan-3-S-carbonsäure
2-[N-(1-S-Carbethoxy-3-phenyl-propyl)-S-lysyl]-cis-endo-2-azabicyclo[3.3.0]octan-3-S-carbonsäure
2-[N-(1-S-Carbethoxy-3-cyclohexyl-propyl)-S-alanyl]-cis-endo-2-azabicyclo[3.3.0]octan-3-S-carbonsäure
2-[N-(1-S-Carboxy-3-cyclohexyl-propyl)-S-alanyl]-cis-endo-2-azabicyclo[3.3.0]octan-3-S-carbonsäure
2-[N-(1-S-Carbethoxy-butyl)-S-alanyl]-cis-endo-2-azabicyclo[3.3.0]octan-3-S-carbonsäure
2-[N-(1-S-Carbethoxy-3-(3,4-dimethoxyphenylpropyl)-S-alanyl]-cis-endo-2-azabicyclo[3.3.0]octan-3-S-carbonsäure
2-[N-(1-S-Carbethoxy-3-cyclopentyl-propyl)-S-alanyl]-cis-endo-azabicyclo-[3.3.0]octan-3-S-carbonsäure
2-[N-(1-S-Carbethoxy-3-phenyl-propyl)-O-methyl-S-tyrosyl]-cis-endo-2-azabicyclo[3.3.0]octan-3-S-carbonsäure
2-[N-(1-S-Carbethoxy-3-phenyl-propyl)-O-ethyl-S-tyrosyl]-cis-endo-2-azabicyclo[3.3.0]octan-3-S-carbonsäure
2-[N-(1-S-Carbethoxy-3-(4-fluorphenyl-propyl)-S-alanyl]-cis-endo-2-azabicyclo[3.3.0]octan-3-S-carbonsäure
2-[N-(1-S-Carbethoxy-3-(4-methoxyphenyl-propyl)-S-alanyl]-cis-endo-2-azabicyclo[3.3.0]octan-3-S-carbonsäure
1-[N-(1-S-Carbethoxy-3-phenyl-propyl)-S-lysyl]-(2S,3aR,6aS)-octahydrocyclopenta[b]pyrrol-2-carbonsäure
1-[N-(1-S-Carbethoxy-3-cyclohexylpropyl)-lysyl]-(2S,3aR, 6aS)-octahydrocyclopenta[b]pyrrol-2-carbonsäure
1-[N-(1-S-Carbethoxy-3-phenyl-propyl)-O-ethyl-S-tyrosyl]-(2S,3aR,6aS)-octahydrocyclopenta[b]pyrrol-2-carbonsäure

1-[N-(1-S-Carbethoxy-3-phenyl-propyl)-S-alanyl]-2-(2S,3aR, 6aS)-octahydrocyclopenta[b]pyrrol-2-carbonsäure
2-[N-(1-S-Carbethoxy-3-phenyl-propyl)-S-alanyl]-2-azaspiro[4.5]decan-3-S-carbonsäure
2-[N-(1-S-Carbethoxy-3-phenyl-propyl)-O-ethyl-2-tyrosyl]-2-azaspiro-[4.5]decan-3-S-carbonsäure
2-[N-(1-S-Carbethoxy-3-phenyl-propyl)-S-lysyl]-2-azaspiro[4.5]decan-3-S-carbonsäure
2-[N-(1-S-Carbethoxy-3-cyclohexylpropyl)-S-alanyl]-2-azaspiro[4.5]decan-3-S-carbonsäure
2-[N-(1-S-Carbethoxy-3-cyclohexylpropyl)-S-lysyl]-2-azaspiro[4.5]decan-3-S-carbonsäure
2-[N-(1-S-Carbethoxy-3-phenyl-propyl)-S-alanyl]-2-azaspiro[4.4]nonan-3-S-carbonsäure
2-[N-(1-S-Carbethoxy-3-phenyl-propyl)-O-ethyl-S-tyrosyl]-2-azaspiro[4.4]nonan-3-S-carbonsäure
2-[N-(1-S-Carbethoxy-3-phenyl-propyl)-S-lysyl]-2-azaspiro[4.4]nonan-3-S-carbonsäure
2-[N-(1-S-Carbethoxy-3-cyclohexyl-propyl)-S-alanyl]-2-azaspiro[4.4]nonan-3-S-carbonsäure
2-[N-(1-S-Carbethoxy-3-cyclopentyl-propyl)-S-alanyl]-2-azaspiro[4.4]nonan-3-S-carbonsäure
2-[N-(1-S-Carbethoxy-3-cyclopentyl-propyl)-S-lysyl]-2-azaspiro[4.4]nonan-3-S-carbonsäure
1'-[N-(1-S-Carbethoxy-3-phenyl-propyl)-S-alanyl]-spiro[bicyclo[2.2.1]heptan-2,3'-pyrrolidin]-5'-S-carbonsäure
1'-[N-(1-S-Carbethoxy-3-phenyl-propyl)-O-ethyl-S-tyrosyl]-spiro[bicyclo[2.2.1]heptan-2,3'-pyrrolidin]-5'-S-carbonsäure
1'-[N-(1-S-Carbethoxy-3-phenyl-propyl)-S-lysyl]-spiro[bicyclo[2.2.1]hepten-2,3'-pyrrolidin]-5'-S-carbonsäure
1'-[N-(1-S-Carbethoxy-3-cyclohexyl-propyl)-S-alanyl]-spiro[bicyclo[2.2.1]heptan-2,3'-pyrrolidin]5'-S-carbonsäure
1'-[N-(1-S-Carbethoxy-3-cyclohexyl-propyl)-S-lysyl]-spiro[bicyclo[2.2.1]hepten-2,3'-pyrrolidin]-5'-S-carbonsäure
1'-[N-(1-S-Carbethoxy-3-phenyl-propyl)-S-alanyl]-spiro-[bicyclo[2.2.2]octan-2,3'-pyrrolidin]-5'-S-carbonsäure
1'-[N-(1-S-Carbethoxy-3-phenyl-propyl)-O-ethyl-tyrosyl]-spiro-[bicyclo[2.2.2]octan-2,3'-pyrrolidin]-5'-S-carbonsäure
1'-[N-(1-S-Carbethoxy-3-phenyl-propyl)-S-lysyl]-spiro[bicyclo[2.2.2]octan-2,3'-pyrrolidin]-5'-S-carbonsäure
1'-[N-(1-S-Carbethoxy-3-cyclohexyl-propyl)-S-alanyl]-spiro-[bicyclo[2.2.2]octan-2,3'-pyrrolidin]-5'-S-carbonsäure
2-[N-(1-S-Carbethoxy-3-phenyl-propyl)-S-alanyl]-2-azatricyclo[4.3.0.1$^{6,9}$]decan-3-S-carbonsäure
2-[N-(1-S-Carbethoxy-3-phenyl-propyl)-O-ethyl-S-tyrosyl]-2-azatricyclo[4.3.0.1$^{6,9}$]decan-3-S-carbonsäure
2-[N-(1-S-Carbethoxy-3-phenyl-propyl)-S-lysyl]-2-azatricyclo[4.3.0.1$^{6,9}$]decan-3-S-carbonsäure
2-[N-(1-S-Carbethoxy-3-cyclohexyl-propyl)-S-alanyl]-2-azatricyclo[4.3.0.1$^{6,9}$]decan-3-S-carbonsäure
2-[N-(1-S-Carbethoxy-3-cyclohexyl-propyl)-S-lysyl]-2-azatricyclo[4.3.0.1$^{6,9}$]decan-3-S-carbonsäure
1-[N-(1-S-Carbethoxy-3-phenyl-propyl)-S-alanyl]-decahydrocyclohepta[b]pyrrol-2-S-carbonsäure
1-[N-(1-S-Carbethoxy-3-phenyl-propyl)-O-ethyl-S-tyrosyl]-decahydrocyclohepta[b]pyrrol-2-S-carbonsäure
1-[N-(1-S-Carbethoxy-3-phenyl-propyl)-S-lysyl]-decahydrocyclohepta[b]pyrrol-2-S-carbonsäure
1-[N-(1-S-Carbethoxy-3-cyclohexyl-propyl)-S-alanyl]-decahydrocyclohepta[b]pyrrol-2-S-carbonsäure
1-[N-(1-S-Carbethoxy-3-cyclohexyl-propyl)-S-lysyl]-decahydrocyclohepta[b]pyrrol-2-S-carbonsäure
2-[N-(1-S-Carbethoxy-3-phenyl-propyl)-S-alanyl]-trans-octahydroisoindol-1-S-carbonsäure
2-[N-(1-S-Carbethoxy-3-phenyl-propyl)-S-alanyl]-cis-octahydroisoindol-1-S-carbonsäure
2-[N-(1-S-Carbethoxy-3-cyclohexyl-propyl)-S-alanyl]-trans-octahydroisoindol-1-S-carbonsäure
2-[N-(1-S-Carbethoxy-3-cyclohexyl-propyl)-S-alanyl]-cis-octahydroisoindol-1-S-carbonsäure
2-[N-(1-S-Carbethoxy-3-phenyl-propyl)-S-alanyl]-cis-octahydrocyclopenta[c]pyrrol-1-S-carbonsäure
2-[N-(1-S-Carbethoxy-3-cyclohexyl-propyl)-S-alanyl]-cis-octahydrocyclopenta[c]pyrrol-1-S-carbonsäure-benzylester
2-[N-(1-S-Carbethoxy-3-cyclohexyl-propyl)-S-lysyl]-cis-octahydrocyclopenta[c]pyrrol-1-S-carbonsäure
1-[N-(1-S-Carbethoxy-3-phenyl-propyl)-S-alanyl]-2,3,3a,4, 5,7a-hexahydroindol-cis-endo-2-S-carbonsäure
1-[N-(1-S-Carbethoxy-3-phenyl-propyl)-S-lysyl]-2,3,3a,4, 5,7a-hexahydroindol-cis-endo-2-S-carbonsäure
2-[N-(1-S-Carbethoxy-3-cyclohexyl-propyl)-S-lysyl]-2-azabicyclo[3.1.0]hexan-3-S-carbonsäure
2-[N-(1-S-Carboxy-3-phenyl-propyl)-S-lysyl]-2-azabicyclo[3.1.0]hexan-cis-endo-3-S-carbonsäure
2-[N-(1-S-Carbethoxy-3-cyclopentylpropyl)-S-alanyl]-2-azabicyclo[3.1.0]hexan-3-carbonsäure
2-[N-(1-S-Carbethoxy-3-phenyl-propyl)-S-alanyl]-cis-endo-2-azabicyclo[3.1.0]hexan-3-S-carbonsäure
2-[N-(1-S-Carbethoxy-3-cyclohexyl-propyl)-S-alanyl]-cis-endo-2-azabicyclo[3.1.0]hexan-3-S-carbonsäure
1'-[N-(1-S-Carbethoxy-3-phenylpropyl)-S-alanyl]-(3'S,5'S)-spiro-bicyclo[2.2.2]-octan-2,3'-pyrrolidin-5'-carbonsäure
1'-[N-(1-S-Carbethoxy-3-phenylpropyl)-S-alanyl]-(3'R,5'S,)-spiro-bicyclo[2.2.2]octan-2,3'-pyrrolidin-5'-carbonsäure
1'-[N-(1-S-Carbethoxy-3-phenylpropyl)-S-alanyl]-(3'S,5'R)-spiro-bicyclo[2.2.2]octan-2,3'-pyrrolidin-5'-carbonsäure

1'-[N-(1-S-Carbethoxy-3-phenylpropyl)-S-alanyl]-(3'R,5'R)-spiro-bicyclo[2.2.2]octan-2,3'-pyrrolidin-5'-carbonsäure
1'-[N-(1-R-Carbethoxy-3-phenylpropyl)-S-alanyl]-(3'S,5'R)-spiro-bicyclo[2.2.2]octan-2,3'-pyrrolidin-5'-carbonsäure
1'-[N-(1-R-Carbethoxy-3-phenylpropyl)-S-alanyl]-(3'S,5'S)-spiro-bicyclo[2.2.2]octan,2,3'-pyrrolidin-5'-carbonsäure
1'-[N-(1-R-Carbethoxy-3-phenylpropyl)-S-alanyl]-(3'R,5'S)-spiro-bicyclo[2.2.2]octan-2,3'-pyrrolidin-5'-carbonsäure
1'-[N-(1-R-Carbethoxy-3-phenylpropyl)-S-alanyl]-(3'R,5'R)-spiro-bicyclo[2.2.2]octan-2,3'-pyrrolidin-5'-carbonsäure
1'-[N-(1-S-Carbethoxy-3-phenylpropyl)-R-alanyl]-(3'S,5'S)-spiro-bicyclo[2.2.2]octan-2,3'-pyrrolidin-5'-carbonsäure
1'-[N-(1-S-Carbethoxy-3-phenylpropyl)-R-alanyl]-(3'R,5'S)-spiro-bicyclo[2.2.2]octan-2,3'-pyrrolidin-5'-carbonsäure
1'-[N-(1-S-carbethoxy-3-phenylpropyl)-R-alanyl]-(3'S,5'R)-spiro-bicyclo[2.2.2]octan-2,3'-pyrrolidin-5'-carbonsäure
1'-[N-(1-S-Carbethoxy-3-phenylpropyl)-R-alanyl]-(3'R,5'R)-spiro-bicyclo[2.2.2]octan-2,3'-pyrrolidin-5'-carbonsäure
1'-[N-(1-R-Carbethoxy-3-phenylpropyl)-R-alanyl]-(3'S,5'S)-spiro-bicyclo[2.2.2]octan-2,3'-pyrrolidin-5'-carbonsäure
1'-[N-(1-R-Carbethoxy-3-phenylpropyl)-R-alanyl]-(3'R,5'S)-spiro-bicyclo[2.2.2]octan-2,3'-pyrrolidin-5'-carbonsäure
1'-[N-(1-R-Carbethoxy-3-phenylpropyl)-R-alanyl]-(3'S,5'R)-spiro-bicyclo[2.2.2]octan-2,3'-pyrrolidin-5'-carbonsäure
1'-[N-(1-R-Carbethoxy-3-phenylpropyl)-R-alanyl]-(3'R,5'R)-spiro-bicyclo[2.2.2]octan-2,3'-pyrrolidin-5'-carbonsäure

Diese Verbindungen lassen sich z.B. nach dem in der deutschen Patentanmeldung P 33 33 455.2 beschriebenen Verfahren herstellen, indem die in der Anmeldung beschriebenen tert. Butyl- oder Benzylderivate in bekannter Weise durch saure oder alkalische Hydrolyse oder durch edelmetallkatalysierte Hydrogenolyse in die Monocarbonsäurederivate überführt werden. Die $N^{\epsilon}$-Benzyloxycarbonylschutzgruppe der Lysinderivate wird durch Edelmetallkatalysierte Hydrogenolyse entfernt. Die oben aufgeführten Verbindungen lassen sich leicht mit physiologisch verträglichen Säuren oder Basen (im Falle von Mono- oder Dicarbonsäuren) in die entsprechenden Salze (z.B. Hydrochloride, Maleinate, Fumarate, etc.) überführen und als Salze die erfindungsgemäße Verwendung finden.

Die Verbindungen der Formel II sind Hemmstoffe des Angiotensin-Converting-Enzymes(ACE) beziehungsweise Zwischenprodukte bei der Herstellung von solchen Hemmstoffen und können auch zur Bekämpfung des Bluthochdrucks verschiedener Genese eingesetzt werden. Die Verbindungen der Formel II und Verfahren zu deren Herstellung sind teilweise bekannt, beispielsweise aus US-Patent 4 129 571, US-Patent 4 374 829, EP-A-79522, EP-A-79022, EP-A-49658, EP-A-51301, US-Patent 4 454 292, US-Patent 4 374 847, EP-A-72352, US-Patent 4 350 704, EP-A-50800, EP-A-46953, US-Patent 4 344 949, EP-A-84164, US-Patent 4 470 972, EP-A-65301 und EP-A-52991. Neue Verbindungen der Formel II werden in analoger Weise hergestellt.

Vorteilhaft sind auch oral wirksame ACE-Inhibitoren (Wirkstoffe z.T. schon oben erwähnt) wie z.B. Ramipril, Enalapril(f), Pentopril(a), Lisinopril(g), Cilazapril(o), RHC 3659, CGS 13945, CGS 13928C(1), CGS 14824A(h), CI-906(j), Alacepril CI-925(k), CV 3317(m), Indoapril(h), Perindopril(i), und andere. Oral wirksame ACE-Inhibitoren sind beispielsweise in Brunnder et al., J. Cardiovasc. Pharmacol. 7 (Suppl. I) 1985 S2-S11 beschrieben.

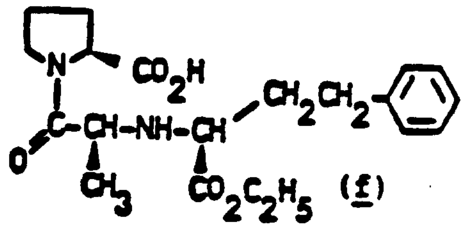

(f)

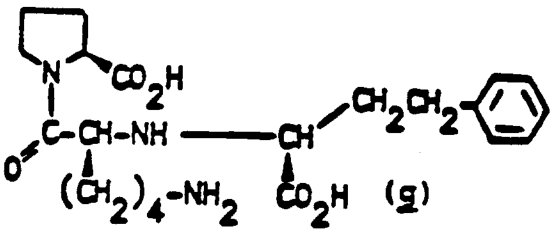

(g)

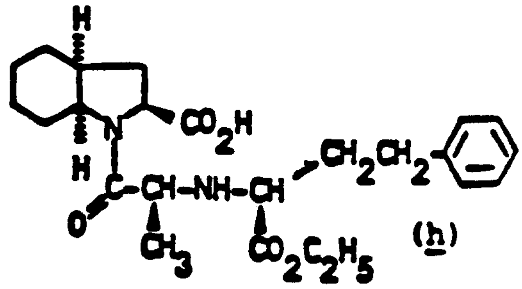

(h)

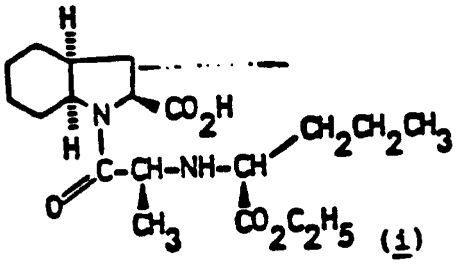

(i)

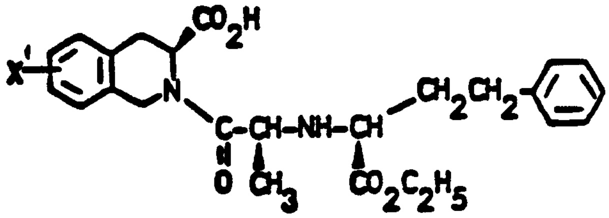

X' = H (j)

X' = 3,4 $OCH_3$ (k)

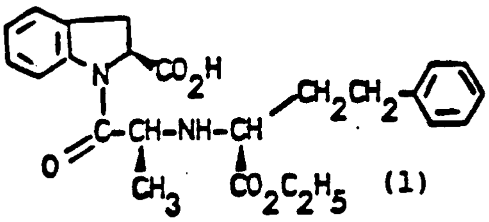

(l)

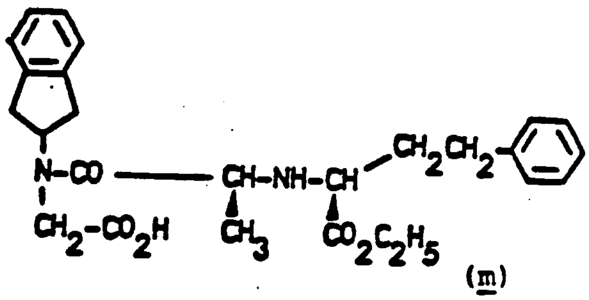

(m)

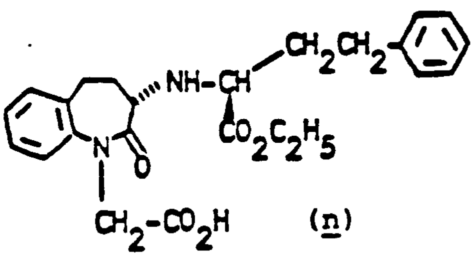

(n)

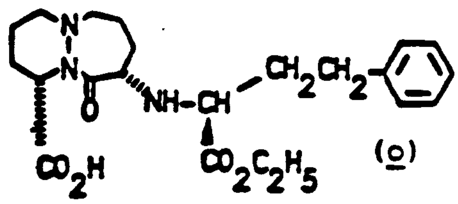

(o)

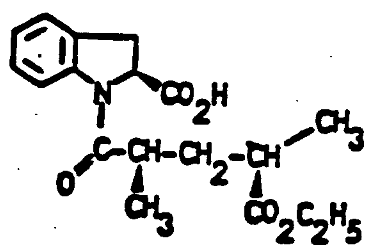

(q)

Bevorzugt sind die aus der EP-A-79022 bekannten ACE-Inhibitoren der Formel III

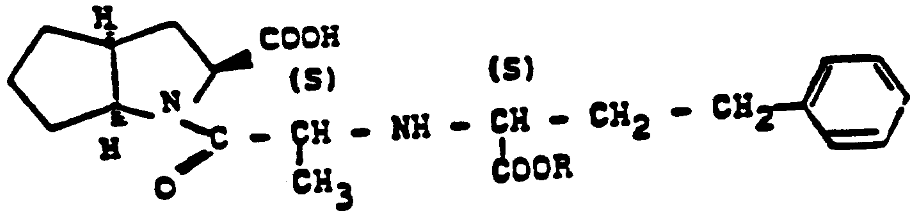

(III)

in welcher
R Wasserstoff, Methyl, Ethyl oder Benzyl
bedeutet,
insbesondere die Verbindung der Formel III, worin R = Ethyl bedeutet (Ramipril).

Weiterhin bevorzugt sind die aus der EP-A-84164 bekannten ACE-Inhibitoren der Formel IV

(IV)

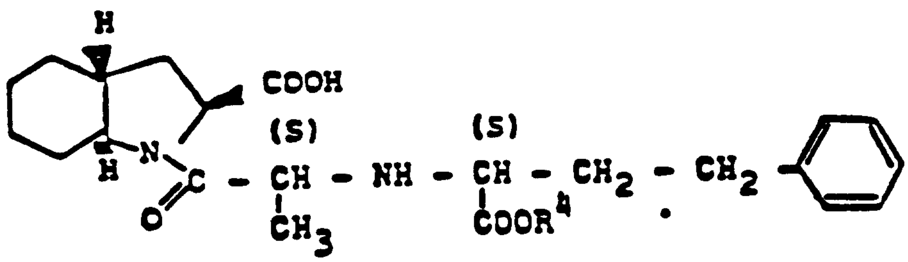

in welcher
$R^4$ Wasserstoff, $(C_1-C_4)$-Alkyl oder Benzyl bedeutet, insbesondere die Verbindung der Formel IV, worin $R^4$ = Ethyl bedeutet.

Bevorzugt sind darüberhinaus
1'-[N-(1-S-Carboethoxy-3-phenyl-propyl)-S-alanyl]-exo- bzw. endo-spirobicyclo[2.2.2]octan-2,3'-pyrrolidin-5'-yl-carbonsäure und Isomere und (S,S,S)-1-Methyl-2(1-carbethoxy-3-phenyl-propyl)-2H-undecahydrocyclopenta[4.5]pyrrolo[1,2-a]-pyrazin-3,8-dion.

Die Kapillarstruktur der Blutgefäße des Gehirns unterscheidet sich von derjenigen in anderen Bereichen des Körpers. Die Gehirnkapillaren sind mit einer Schicht von Endothelzellen umgeben, welche besonders eng (durch "tight junctions") miteinander verknüpft sind. Gehirnkapillaren besitzen außerdem sehr viel weniger jener Poren, durch welche in anderen Blutkapillaren die niedermolekularen Substanzen in das umgebende Gewebe hinein oder aus diesem heraustreten können. Auf diese Weise erhält bei den Gehirnkapillaren die Eigenschaft der Lipialöslichkeit eine sehr viel größere Bedeutung für die Verteilung zwischen Blut und umgebendem Gewebe, als dies für den Restkörper der Fall ist.

Es sind daher Verbindungen der Formel II bevorzugt, in welcher wenigstens einer der Reste R, $R^1$, $R^2$ und $R^3$ für einen lipophilen Rest, wie einen langkettigen aliphatischen, alicyclisch-aliphatischen, araliphatischen oder heteroaraliphatischen Rest, einen genügend großen alicyclischen Rest oder ein entsprechend substituierten alicyclischen, aromatischen oder heteroaromatischen Rest steht, oder einen solchen als Teilstruktur enthält.

Die Erfindung betrifft weiterhin ein Verfahren zur Herstellung einer Verbindung der Formel II, das dadurch gekennzeichnet ist, daß man ihre Fragmente in einem geeigneten Lösungsmittel gegebenenfalls in Gegenwart einer Base und/oder eines Kupplungshilfsmittels miteinander umsetzt, gegebenenfalls intermediär entstehende ungesättigte Verbindungen, wie Schiffsche Basen, reduziert, zum Schutz reaktiver Gruppen temporär eingeführte Schutzgruppen abspaltet, Verbindungen der Formel II mit freier(en) Carboxylgruppe(n) gegebenenfalls verestert und die erhaltenen Verbindungen gegebenenfalls in ihre physiologisch verträglichen Salze überführt.

In der genannten Weise kann man beispielsweise Verbindungen der Formel V mit Verbindungen der Formel VI umsetzen.

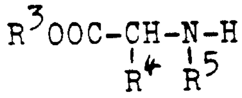

(V)

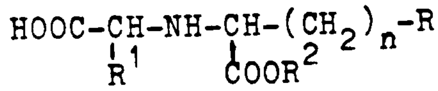

(VI)

Die Umsetzung dieser Verbindungen kann beispielsweise in Analogie zu bekannten Peptidkupplungsverfahren in einem organischen Lösungsmittel wie DMF, $CH_2Cl_2$, DMA in Gegenwart von Kupplungshilfsmitteln, wie Carbodiimiden (z.B. Dicyclohexylcarbodiimid), Diphenylphosphorylazid, Alkanphosphorsäureanhydriden, Dialkylphosphinsäureanhydriden oder N,N-Succinimidylcarbonate in einem Lösungsmittel wie $CH_3CN$ durchgeführt werden. Aminogruppen in Verbindungen der Formel V können mit Tetraethyldiphosphit aktiviert werden. Die Verbindungen der Formel VI können in Aktivester (z.B. mit 1-Hydroxybenzotriazol), gemischte Anhydride (z.B. mit Chlorameisensäureestern), Azide oder Carbodiimid-Derivate überführt und damit aktiviert werden (vgl. Schröder, Lübke, The Peptides, Band 1, New York 1965, Seiten 76 - 136). Die Reaktion wird vorzugsweise zwischen -20 °C und dem Siedepunkt des Reaktionsgemisches durchgeführt.

Ebenso lassen sich auch Verbindungen der Formel VII mit Verbindungen der Formel VIII unter Bildung von Verbindungen der Formel II umsetzen,

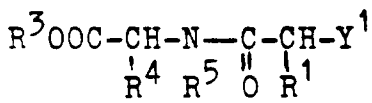

(VII)

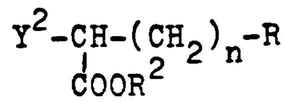

(VIII)

worin entweder $Y^1$ für Amino und $Y^2$ für eine Abgangsgruppe oder $Y^1$ für eine Abgangsgruppe und $Y^2$ für Amino stehen. Geeignete Abgangsgruppen sind z.B. Cl, Br, I, Alkylsulfonyloxy oder Arylsulfonyloxy.

Alkylierungen dieser Art führt man zweckmäßigerweise in Wasser oder einem organischen Lösungsmittel wie einem niedrigen aliphatischen Alkohol (wie Ethanol), Benzylalkohol, Acetonitril, Nitromethan oder Glycolether bei einer Temperatur zwischen -20 °C und dem Siedepunkt des Reaktionsgemisches in Gegenwart einer Base wie einem Alkalimetallhydroxid oder einem organischen Amin durch.

Des weiteren lassen sich Verbindungen der Formel IX mit Verbindungen der Formel X kondensieren,

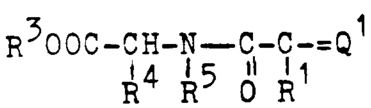

(IX)

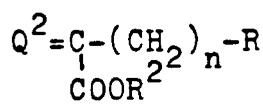

(X)

worin entweder $Q^1$ für Amino + Wasserstoff und $Q^2$ für Oxo steht oder $Q^1$ für Oxo und $Q^2$ für Amino + Wasserstoff steht. Die Kondensation wird zweckmäßigerweise in Wasser oder einem organischen Lösungsmittel, wie einem niederen aliphatischen Alkohol, bei einer Temperatur zwischen -20 °C und dem Siedepunkt des Reaktionsgemisches in Gegenwart eines Reduktionsmittels, wie $NaBH_3CN$, durchgeführt, wobei Verbindungen der Formel I direkt erhalten werden. Man kann aber auch die als Zwischenprodukte entstehenden Schiff'schen Basen oder Enamine gegebenenfalls nach vorheriger Isolierung unter Bildung von Vorbindungen der Formel II reduzieren, beispielsweise durch Hydrierung in Gegenwart eines Übergangmetallkatalysators.

Schließlich führt auch die Umsetzung von Verbindungen der Formel IX ($Q^1 = H + NH_2$) mit Verbindungen der Formel XI oder deren Umsetzung mit Verbindungen der Formeln XII und XIII zweckmäßig in Gegenwart einer Base, wie Natriumalkoholat, in einem organischen Lösungsmittel, wie einem niederen

Alkohol, bei einer Temperatur zwischen -10 °C und dem Siedepunkt des Reaktionsgemisches zu Verbindungen der Formel II (n = 2),

$R^2OOC\text{-}CH = CH\text{-}COR$ (XI)

$OCH\text{-}COOR^2$ (XII)

$R\text{-}CO\text{-}CH_3$ (XIII)

wobei intermediär entstehende Schiff'sche Basen wie oben beschrieben reduziert und eine Carbonylgruppe reduktiv (beispielsweise mit einem komplexen Hydrid) in Methylen überführt werden.

In den obengenannten Formeln V - XIII sind R -$R^5$ und n wie in Formel II definiert. Temporär zum Schutz nicht an der Reaktion beteiligter reaktiver Gruppen eingeführte Schutzgruppen werden nach beendeter Reaktion in an sich bekannter Weise abgespalten (vgl. Schröder, Lübke, loc cit., Seiten 1 - 75 und 246 - 270; Greene, "Protective Groups in Organic Synthesis", New York 1981).

Die Herstellung der neuen Verbindungen der allgemeinen Formel I oder II kann beispielsweise auch unter Anwendung dem Fachmann vertrauter Veresterungsmethoden erfolgen (siehe z.B. Buchler, Pearson, Survey of Organic Syntheses, Vol. 1, New York 1970, S. 802 - 825; Houben-Weyl, Methoden der Organischen Chemie, Band E5, 1985, S. 656 - 773).

a) Die Umsetzung einer Mono- oder Dicarbonsäure der allgemeinen Formel I oder II, worin mindestens einer der Reste $R^2$ und $R^3$ Wasserstoff bedeutet, mit einem entsprechenden Alkohol unter saurer Katalyse (Mineralsäure oder saurer Ionenaustauscher).

b) Die Alkylierung einer Mono- oder Dicarbonsäure der allgemeinen Formel I oder II, worin mindestens einer der Reste $R^2$ und $R^3$ Wasserstoff bedeutet, mit einer Verbindung $R^2Z$ oder $R^3Z$, worin Z eine nukleophil zu verdrängende Abgangsgruppe (wie Halogen, Tosylat) bedeutet, in einem polaren protischen oder dipolaren aprotischen Lösungsmittel in Anwesenheit einer Base wie Alkalimetallhydroxid oder -alkoholat.

c) Die Umsetzung einer Mono- oder Dicarbonsäure der allgemeinen Formel I oder II, worin mindestens einer der Reste $R^2$ und $R^3$ Wasserstoff bedeutet, mit einem Diazoalken in einem inerten organischen Lösungsmittel wie $CH_2Cl_2$.

Die nootrope Wirkung der erfindungsgemäßen Verbindungen wurde an Mäusen, die ein Körpergewicht von 20 - 25 g besaßen im inhibitory (passive) avoidance test (step-through-Modell) geprüft. Eine modifizierte Form der von J. KOPP, Z. BODANECKY und M.E. JARVIK beschriebenen Testmethode wurde von J. BURES, O. BURESOVA und J. HUSTON in "Techniques and Basic Experiments for the Study of Brain and Behavior", Elsevier Scientific Publishers, Amsterdam (1983) beschrieben.

Entsprechend diesen Literaturangaben wird eine Substanz dann als nootrop wirksam bezeichnet, wenn sie bei den Versuchstieren die mittels eines elektroconvulsiven Schocks erzeugte Amnesie oder die mittels Scopolamin induzierte Amnesie aufzuheben vermag.

Die Versuche wurden nach modifizierten Testmethoden durchgeführt. Als Vergleichsverbindung diente das bekannte Nootropikum 2-Oxo-1-pyrrolidinylessigsäureamid (Piracetam). Die deutliche Überlegenheit der erfindungsgemäßen Verbindungen über die Vergleichssubstanz zeigte sich darin, daß die Scopolamininduzierte Amnesie im inhibitory avoidance-Test sich mit einer MED (minimal effective Dosis) von 1.0 - 30 mg/kg p.o. aufheben läßt. Die Vergleichssubstanz hat eine MED von ca. 500 - 1000 mg/kg p.o.

Die erfindungsgemäßen Verbindungen haben meist nur eine geringe Toxizität.

Die erfindungsgemäßen Verbindungen sind aufgrund ihrer pharmakologischen Eigenschaften neben der Behandlung des Bluthochdruckes auch für die Behandlung cognitiver Dysfunktionen unterschiedlicher Genese, wie sie z.B. der Alzheimer'schen Krankheit oder der senilen Demenz auftreten, geeignet.

Die Erfindung betrifft daher weiter die Anwendung der erfindungsgemäßen Verbindungen bei der Behandlung und Prophylaxe cognitiver Dysfunktionen bei Bluthochdruck-Patienten.

Die Erfindung umfaßt weiterhin die genannten Verbindungen enthaltende Arzneimittel, Verfahren zu deren Herstellung sowie die Verwendung der erfindungsgemäßen Verbindungen bei der Herstellung von Arzneimitteln, die zur Behandlung und Prophylaxe der vorstehend genannten Krankheiten eingesetzt werden.

In Ausübung der erfindungsgemäßen Methode können die oben beschriebenen Angiotensin-Converting-Enzyme-Inhibitoren an Säugern wie Affen, Hunden, Katzen, Ratten, Menschen etc. angewendet werden.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfsstoffen in Form

von Tabletten, Draages, Kapseln, Suppositorien, Emulsionen, Suspensionen oder Lösungen eingesetzt, wobei der Wirkstoffgehalt bis etwa 95%, vorteilhafterweise zwischen 10 und 75%, beträgt.

Welche Hilfsstoffe für die gewünschte Arzneimittelformulierung geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können beispielsweise oral, rektal oder parenteral (z.B. intravenös oder subcutan) appliziert werden, wobei die orale Applikation bevorzugt ist.

Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Darreichungsformen gebracht wie Tabletten, Dragees, Steckkapseln, wässrige, alkoholische oder ölige Suspensionen oder wässrige, alkoholische oder ölige Lösungen. Als inerte Träger können z.B. Gummi arabicum, Magnesia, Magnesiumcarbonat, Milchzucker, Glucose oder Stärke, insbesondere Maisstärke verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht wie Sonnenblumenöl oder Lebertran.

Zur subcutanen oder intravenösen Applikation werden die aktiven Verbindungen oder deren physiologisch verträglicher Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage Wasser, physiologische Kochsalzlösung oder Alkohole, z.B. Ethanol, Propanol, Glycerin, daneben auch Zuckerlösungen wie Glucose- oder Mannitlösungen oder auch eine Mischung aus den verschiedenen genannten Lösungsmitteln.

Die folgenden Beispiele 1 - 6 geben die Anwendungsformen zur Prophylaxe und Behandlung cognitiver Dysfunktionen nach der erfindungsgemäßen Methode an. Die erfindungsgemäßen Verbindungen können analog den Beispielen in die entsprechenden Anwendungsformen gebracht werden.

Beispiel 1

Herstellung des erfindungsgemäß verwendeten Mittels zur oralen Anwendung in der Behandlung und Prophylaxe cognitiver Dysfunktionen.

1000 Tabletten, die je 10 mg 2-[N-(1-S-Carbethoxy-3-phenyl-propyl)-S-alanyl]-1S,3S,5S-2-azabicyclo-[3.3.0]octan-3-carbonsäure enthalten, werden mit den folgenden Hilfsmitteln hergestellt:

| | |
|---|---|
| 2-[N-(1-S-Carbethoxy-3-phenyl-propyl)-S-alanyl]-(1S,3S,5S)-2-azabicyclo-[3.3.0]octan-3-carbonsäure | 10 g |
| Maisstärke | 140 g |
| Gelatine | 7,5 g |
| Mikrokristalline Cellulose | 2,5 g |
| Magnesiumstearat | 2,5 g |

2-[N-(1-S-Carbethoxy-3-phenyl-propyl)-S-alanyl]-(1S,3S, 5S)-2-azabicyclo[3.3.0]octan-3-carbonsäure und Maisstärke werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das entstandene Granulat wird zu 1000 Tabletten gepreßt, wobei jede Tablette 10 mg des ACE-Hemmers enthält.
Diese Tabletten können zur Behandlung und Prophylaxe cognitiver Dysfunktionen verwendet werden.

Beispiel 2

Analog Beispiel 1 werden 1000 Tabletten hergestellt, die je 10 mg 1'-[N-(1-S-Carbethoxy-3-phenylpropyl)-S-alanyl]-(3'S,5'S)-Spirobicyclo[2.2.2]octan-2,3'-pyrrolidin-5'-carbonsäure enthalten.

Beispiel 3

Gelatine-Kapseln, die je 10 mg 1'-[N-(1-S-Carbethoxy-3-phenylpropyl)-S-alanyl]-(3'R,5'S)-spirobicyclo-[2.2.2]octan-2,3'-pyrrolidin-5'-carbonsäure enthalten, werden mit der folgenden Mischung gefüllt:

| | |
|---|---|
| 1'-[N-(1-S-Carbethoxy-3-phenylpropyl)-S-alanyl]-(3'R,5'S)-spirobicyclo[2.2-.2]octan-2,3'-pyrrolidin-5'-carbonsäure | 10 mg |
| Magnesiumstearat | 1 mg |
| Lactose | 214 mg |

Diese Kapseln können zur Behandlung und Prophylaxe cognitiver Dysfunktionen verwendet werden.

Beispiel 4

Die Herstellung einer Injektionslösung wird im folgenden beschrieben:

| | |
|---|---|
| 2-[N-(1-S-Carboxy-3-phenyl-propyl)-S-alanyl]-(1S,3S,5S)-2-azabicyclo[3.3.0]octan-3-carbonsäure | 250 mg |
| Methylparaben | 5 g |
| Propylparaben | 1 g |
| Natriumchlorid | 25 g |
| Wasser für Injektion | 5 l |

2-[N-(1-S-Carboxy-3-phenyl-propyl)-S-alanyl]-(1S,3S,5S)-2-azabicyclo[3.3.0]octan-3-carbonsäure, die Konservierungsstoffe und Natriumchlorid werden in 3 l Wasser für Injektion gelöst und auf 5 l mit Wasser für Injektion aufgefüllt. Die Lösung wird steril gefiltert und aseptisch in vorsterilisierte Flaschen gefüllt, die mit sterilisierten Gummikappen verschlossen werden. Jede Flasche enthält 5 ml Lösung.

Beispiel 5

Tabletten, die zur Behandlung oder Prophylaxe cognitiver Dysfunktionen verwendet werden können, werden wie in Beispiel 1 beschrieben hergestellt, mit der Ausnahme, daß anstelle von 2-[N-(1-S-Carbethoxy-3-phenyl-propyl)-S-alanyl]-(1S,3S,5S)-2-azabicyclo[3.3.0]octan-3S-carbonsäure 2-[N-(1-S-Carboxy-3-phenyl-propyl)-S-alanyl]-(1S,3S,5S)-2-azabicyclo[3.3.0]octan-3-carbonsäure oder 1-[N-(1-S-Carboxy-3-phenyl-propyl)-S-alanyl]-(2S,3aR, 7aS)-octahydroindol-2-carbonsäure oder 1-[N-(1-S-Carbethoxy-3-phenyl-propyl)-S-alanyl]-cis-2,3,3a,-4,5,7a-hexahydro[1H]indol-2-S-endo-carbonsäure oder 1-[N-(1-S-Carboxy-3-phenyl-propyl)-S-alanyl]-cis-2,3,3a,4, 5,7a-hexahydro[1H]indol-2S-endo-carbonsäure oder 2-[N-(1-S-Carboxy-3-phenyl-propyl)-S-lysyl]-(1S,3S,5S)-2-azabicyclo[3.3.0]octan-3-carbonsäure oder 2-[N-(1-S-Carbethoxy-3-cyclohexyl-propyl)-S-alanyl]-(1S,3S, 5S)-2-azabicyclo[3.3.0]octan-3-carbonsäure oder N-(1-S-Carboxy-3-cyclohexyl-propyl)-S-lysyl-(1S,3S,5S)-2-azabicyclo[3.3.0]octan-3-carbonsäure oder 1'-[N-(1-S-Carbethoxy-3-phenyl-propyl)-S-alanyl]-exo-spirobicyclo[2.2.2]octan-2,3'-pyrrolidin-5'-S-yl-carbonsäure oder (S,S,S)-1-Methyl-2-(1-carbethoxy-3-phenyl-propyl)-2H-undecahydro-cyclopenta[4.5]pyrrolo[1,2-a]pyrazin-3,8-dion oder 1'-[N-(1-S-Carbethoxy-3-phenylpropyl)-S-alanyl]-endo-spirobicyclo[2.2.2]octan-2,3'-pyrrolidinyl-5'-S-carbonsäure verwendet werden.

Beispiel 6

Eine Injektionslösung wird analog der in Beispiel 4 beschriebenen Vorschrift hergestellt, mit der Ausnahme, daß anstelle von 2-[N-(1-S-Carbethoxy-3-phenyl-propyl)-S-alanyl]-(1S,3S,5S)-2-azabicyclo[3.3.0]-octan-3-carbonsäure 2-[N-(1-S-Carboxy-3-phenyl-propyl)-S-alanyl]-(1S,3S,5S)-2-azabicyclo[3.3.0]octan-3-carbonsäure oder 1-[N-(1-S-Carbethoxy-3-phenyl-propyl)-S-alanyl]-(2S,3aR, 7aS)-octahydroindol-2-carbonsäurehydrochlorid oder 1-[N-(1-S-Carboxy-3-phenyl-propyl)-S-alanyl]-(2S,3aR,7aS)-octahydroindol-2-carbonsäure oder 1-[N-(1-S-Carbethoxy-3-cyclohexyl-propyl)-S-alanyl]-cis-2,3,3a,4,5,7a-hexahydro[1H]indol-2-S-endo-carbonsäure oder 1-[N-(1-S-Carboxy-3-phenyl-propyl)-S-alanyl]-cis-2,3,3a,4, 5,7a-hexahydro[1H]indol-2-S-endo-carbonsäure oder 2-[N-(1-Carboxy-3-phenyl-propyl)-S-lysyl]-(1S,3S,5S)-2-azabicyclo[3.3.0]octan-3-carbonsäure oder 2-[N-(1-S-Carbethoxy-3-cyclohexyl-propyl)-S-alanyl]-(1S,3S,5S)-2-azabicyclo[3.3.0]-octan-3-carbonsäure oder 2-[N-(1-S-Carboxy-3-cyclohexyl-propyl)-S-lysyl]-(1S,3S,5S)-2-azabicyclo[3.3.0]-octan-3-carbonsäure oder 1'[N-(1-S-Carboxy-3-phenylpropyl)-S-alanyl]-endo-spirobicyclo[2.2.2]octan-2,3'-pyrrolidin-5'-S-carbonsäure 1'[N-(1-S-Carboxy-3-phenylpropyl)-S-alanyl]-exo-spirobicyclo[2.2.2]octan-2,3'-pyrrolidin-5'-S-carbonsäure angewendet werden.

Die nun folgenden Beispiele 7 - 95 sollen das erfindungsgemäße Verfahren zur Herstellung der neuen Verbindungen der Formel I bzw. III erläutern, ohne daß die Erfindung darauf beschränkt wäre.

Beispiel 7:

2-[N-(1S)-Ethoxycarbonyl-3-phenylpropyl)-S-alanyl]-cis, endo-2-azabicyclo[3.3.0]octan-3-S-carbonsäureoctadecylester

23 g des analog EP-A-79 022 hergestellten cis,endo-2-Azabicyclo[3.3.0]octan-3-carbonsäureoctadecylesters werden mit 6,7 g HOBt, 13,8 g N-(1S)-Carbethoxy-3-phenylpropyl)-S-alanin, und 10,2 g Dicyclohexylcarbodiimid in 200 ml Dimethylformamid zur Reaktion gebracht. Nach 3 Stunden Rühren bei Raumtemperatur saugt man von ausgefallenem Dicyclohexylharnstoff ab, engt ein, nimmt in 1 l Essigester auf und schüttelt mit 3 x 500 ml 5-prozentiger $NaHCO_3$-Lösung aus. Die organische Phase wird eingeengt, mit Essigester/Petrolether im Verhältnis 2:1 über eine Säule aus 1 kg Kieselgel chromatographiert und auf diese Weise in die Titelverbindung und die diastereomere (S,S,R)-Verbindung aufgetrennt.

Beispiel 8:

1-[N-(1S)-Dodecyloxycarbonyl-3-phenylpropyl)-S-alanyl]-2S, 3aR,7aR-octahydroindol-2-carbonsäure

a) 1-[N-(1S)-Dodecyloxycarbonyl-3-phenylpropyl)-S-alanyl]-2S, 3aR, 7aR-octahydroindol-2-carbonsäurebenzylester
10 mMol S-Alanyl-2S,3aR,7aR-octahydroindol-2-carbonsäure-benzylester (hergestellt nach EP-A-84 164) werden in 30 ml wasserfreiem Ethanol gelöst. Man stellt die Lösung mittels ethanolischem Kaliumhydroxid auf einen pH-Wert von 7,0 und gibt 1 g pulverisiertes Molekularsieb (4A) und anschließend 10 mMol 2-Keto-4-phenylbuttersäuredodecylester hinzu. Es wird eine Lösung von 1 g Natriumcyanborhydrid in 10 ml wasserfreiem Ethanol langsam zugetropft. Nach einer Reaktionszeit von 20 Stunden bei 20 bis 25 °C wird die Reaktionslösung filtriert und das Lösungsmittel abdestilliert. Der Rückstand wird in Essigester/Wasser aufgenommen. Nach Eindampfen der Essigesterphase wird der Rückstand an Kieselgel mit Essigester/Cyclohexan (1:4) chromatographiert.
b) Die nach a) erhaltene Verbindung wird in Ethanol in Gegenwart von Palladium-Tierkohle (10 %) bei 20-25 °C unter Normaldruck hydriert. Nach Abtrennen des Katalysators wird die Lösung mit 0,5 n ethanolischem Chlorwasserstoff bis zur sauren Reaktion versetzt. Die Lösung wird im Vakuum eingeengt und der Rückstand zur Kristallisation mit Diisopropylether verrieben.

Beispiel 9:

2-[N-[(2S)-Ethoxycarbonyl-3-phenylpropyl]-L-alanyl]-(1S, 3S,5S)-2-azabicyclo[3.3.0]octan-3-carbonsäureisobutylester

2,00 g (4,80 mmol) 2-[N-[(1S)-Ethoxycarbonyl-3-phenylpropyl]-L-alanyl]-(1S,3S,5S)-2-azabicyclo[3.3.0]-octan-3-carbonsäure wurden in 100 ml Isobutanol gelöst, 0,1 ml konzentrierte Schwefelsäure zugesetzt und die Mischung 15 Stunden am Rückfluß gekocht. Nach dem Abkühlen wurde das Lösungsmittel am Rotationsverdampfer abgezogen und der Rückstand in Methylenchlorid aufgenommen. Diese Lösung wurde je einmal mit Wasser, gesättigter wäßriger $NaHCO_3$-Lösung und erneut Wasser gewaschen, über $MgSO_4$ getrocknet, eingeengt und durch Chromatographie an 200 g Kieselgel (Laufmittel Methylenchlorid/Essigester 8:2) von Verunreinigungen abgetrennt.
Ausbeute 0:51% der Theorie öliges Produkt.
$[\alpha]_D^{20}$ = -28,2 ° (c = 1, Methanol).
Dieses Produkt wurde in Ether gelöst, mit gesättigter etherischer Salzsäure auf pH 2 gestellt, das Lösungsmittel abgedampft und der Rückstand aus Diisopropylether kristallisiert.
Daten des Hydrochlorids:
Schmp. 123 - 124 °C
$[\alpha]_D^{20}$ = +17,7 ° (c = 1, Methanol)

Beispiel 10:

2-[N-[(1S)-Ethoxycarbonyl-3-phenylpropyl]-L-alanyl]-(1S,3S,5S)-2-azabicyclo[3.3.0]octan-3-carbonsäurebenzhydrylester

2.07 g (4,97 mmol) 2-[N-[(1S)-Ethoxycarbonyl-3-phenyl-propyl]-L-alanyl]-(1S,3S,5S)-2-azabicyclo[3.3.0]-octan-3-carbonsäure wurden in 50 ml Aceton gelöst und unter Eiskühlung eine Lösung von 1,16 g (5,98 mmol) Diphenyldiazomethan in 50 ml Aceton zugetropft. Anschließend wurde die Lösung 26 Stunden bei Raumtemperatur gerührt, das Lösungsmittel am Rotationsverdampfer abgedampft und der Rückstand durch Flash-Chromatographie an 150 g Kieselgel (Laufmittel Toluol/Ethanol 98:2) gereinigt.
Ausbeute: 2,55 g (88%) öliges Produkt
$[\alpha]_D^{20}$ = -33,8° (c = 1, Methanol).

Beispiel 11:

2-[N-[(1S)-Ethoxycarbonyl-3-phenylpropyl]-L-alanyl]-(1S,3S,5S)-2-azabicyclo[3.3.0]octan-3-carbonsäureoctadecylester

2,08 g (5,00 mmol) 2-[N-[(1S)-Ethoxycarbonyl-3-phenylpropyl]-L-alanyl]-(1S,3S,5S)-2-azabicyclo[3.3.0]-octan-3-carbonsäure wurden in 25 ml absolutem Dimethylformamid gelöst, 1.00 g (10,0 mmol) Kaliumhydrogencarbonat zugesetzt und 90 Minuten bei 40°C gerührt. Nach Abkühlen auf Raumtemperatur wurde eine Lösung von 4,00 g (12,0 mmol) 1-Bromoctadecan in 20 ml absolutem Dimethylformamid zugetropft und 4 Stunden bei 40°C gerührt.
Das Lösungsmittel wurde am Rotationsverdampfer bei ca. 1 Torr abgezogen und der Rückstand zwischen Wasser und Methylenchlorid verteilt. Die organische Phase wurde abgetrennt, über $MgSO_4$ getrocknet und eingeengt. Aus dem Rohprodukt (5,40 g) wurden nach Säulenchromatographie an 200 g Kieselgel (Laufmittel Toluol/Ethanol 99:1) 3,05 g (92 %) des Produktes isoliert.
$[\alpha]_D^{20}$ = -19,6° (c = 1, Methanol)

Beispiel 12:

2-[N-[(1S)-Benzhydryloxycarbonyl-3-phenylpropyl]-L-alanyl]-(1S,3S,5S)-2-azabicyclo[3.3.0]octan-3-carbonsäurebenzylester

a) (2R)-Hydroxy-4-phenylbuttersäurebenzhydrylester
Zu einer Lösung von 7.40 g (41,1 mmol) (2R)-Hydroxy-4-phenylbuttersäure in 200 ml absolutem Aceton wurde unter Eiskühlung während 20 Minuten eine Lösung von 10,1 g (52,1 mmol) Diphenyldiazomethan in 400 ml absolutem Aceton zugetropft und die Reaktionsmischung 20 Stunden bei Raumtemperatur gerührt. Das Lösungsmittel wurde abgedampft und der Rückstand mit 100 ml Petrolether verrieben. Es wurden 6,4 g kristallines Produkt erhalten. Die Mutterlauge wurde eingedampft und durch Säulenchromatographie an 700 g Kieselgel (Laufmittel Toluol/Ethanol 99:1) weitere 6,0 g des Produkts isoliert.
Gesamtausbeute: 12,4 g (87 %).
Schmp. 88 - 89°C.
$[\alpha]_D^{20}$ = -1,8° (c = 5, Methanol).

b) 2-[N-[(1S)-Benzhydryloxycarbonyl-3-phenylpropyl]-L-alanyl]-(1S,3S,5S)-2-azabicyclo[3.3.0]octan-3-carbonsäurebenzylester

$b_1$) 1,80 g (4,33 mmol) 2-(N-tert.butoxycarbonyl-L-alanyl)-(1S,3S,5S)-2-azabicyclo[3.3.0]octan-3-carbonsäurebenzylester wurden in 4,5 ml Trifluoressigsäure gelöst und 90 Minuten bei Raumtemperatur gerührt. Die Reaktionslösung wurde eingedampft und zur Entfernung von Trifluoressigsäureestern dreimal am Rotationsverdampfer mit Toluol abgezogen. Der Rückstand, bestehend aus 1,90 g 2-(L-Alanyl)-(1S,3S,5S)-2-azabicyclo[3.3.0]octan-3-carbonsäurebenzylester-trifluor acetat, wurde in 10 ml absolutem Methylenchlorid gelöst (Lösung A).

$b_2$) 1,63 g (4,71 mmol) (2R)-Hydroxy-4-phenyl-buttersäurebenzhydrylester aus Beispiel 12 a) wurden zusammen mit 0,4 ml absolutem Pyridin in 25 ml absolutem Methylenchlorid gelöst und bei -10°C innerhalb von 20 Minuten 1,41 g (5,00 mmol) Trifluormethansulfonsäureanhydrid zugetropft. Anschließend wurde das Kühlbad entfernt und nach Erreichen von Raumtemperatur das Lösungsmittel abgedampft. Der Rückstand wurde über 50 g Kieselgel mit Methylenchlorid filtriert und eingeengt. Es wurden 1,70 g 4-Phenyl-(2R)-trifluormethylsulfonyloxy-buttersäurebenzhydrylester erhalten und in 10

ml absolutem Methylenchlorid gelöst (Lösung B).

$b_3$) Zu Lösung A wurde 1,0 ml (7,40 mmol) Triethylamin zugesetzt und anschließend bei 0 °C Lösung B langsam zugetropft. Das Kühlbad wurde entfernt und die Reaktionslösung 19 Stunden bei Raumtemperatur gerührt, dann dreimal mit Wasser gewaschen, über $MgSO_4$ getrocknet und eingedampft. Der Rückstand wurde an 80 g Kieselgel (Laufmittel Cyclohexan/Essigester 8:2 und 7:3) chromatographisch gereinigt und 0,95 g (30 %) des gewünschten Produktes erhalten.

Schmp. 81 - 85 °C

$[\alpha]_D^{20}$ = -55,2 ° (c = 1, Methanol)

Durch geeignete Kombinationen der in den voranstehenden Beispielen geschilderten Methoden werden folgende weitere Verbindungen hergestellt (die Bezeichnung der Ringsysteme entspricht derjenigen für die Verbindungen der allgemeinen Formeln I und II):

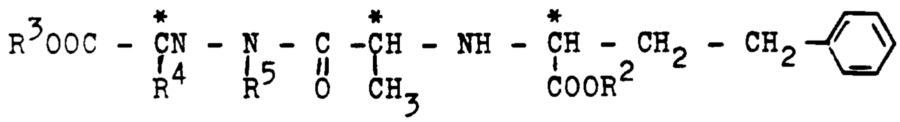

| Bsp. | 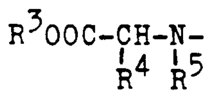 | $R^2$ | $R^3$ |
|---|---|---|---|
| 13 | Ringsystem A | $-C_2H_5$ | $-CH(CH_3)_2$ |
| 14 | Ringsystem B | $-C_2H_5$ | $-CH_2-CH(CH_3)_2$ |
| 15 | Ringsystem C | $-CH_3$ | 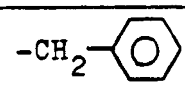 |
| 16 | Ringsystem C | $-C_2H_5$ | $-CH(C_6H_5)_2$ |
| 17 | Ringsystem D | $-C_2H_5$ | 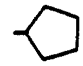 |
| 18 | Ringsystem D | $-C_2H_5$ | 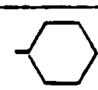 |
| 19 | Ringsystem D | $-C_2H_5$ | 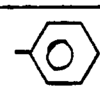 |
| 20 | Ringsystem D | $-C_2H_5$ | 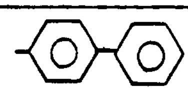 |
| 21 | Ringsystem D | $-C_2H_5$ | 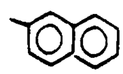 |
| 22 | Ringsystem D | $-C_2H_5$ | 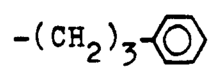 |
| 23 | Ringsystem E | $-C_2H_5$ | $-(CH_2)_5-CH_3$ |
| 24 | Ringsystem F | $-CH_3$ | $-CH(C_6H_5)_2$ |
| 25 | Ringsystem G | $-C_2H_5$ | $-CH(CH_3)_2$ |
| 26 | Ringsystem G | $-C_2H_5$ | $-CH(C_6H_5)_2$ |

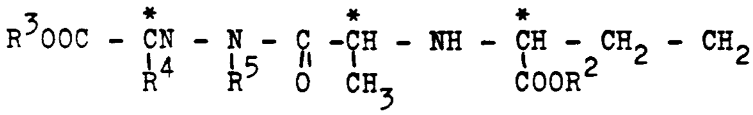

| Bsp. | 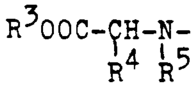 | $R^2$ | $R^3$ |
|---|---|---|---|
| 27 | Ringsystem G | $-C_2H_5$ | $-(CH_2)_2-$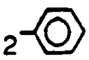 |
| 28 | Ringsystem H | $C_2H_5$ | $-CH(CH_3)_2$ |
| 29 | Ringsystem H | $C_2H_5$ | $-CH_2-CH(CH_3)_2$ |
| 30 | Ringsystem H | $C_2H_5$ | $-(CH_2)_5-CH_3$ |
| 31 | Ringsystem H | $C_2H_5$ | 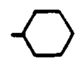 |
| 32 | Ringsystem H | $C_2H_5$ | 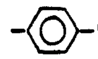$-CH_3$ |
| 33 | Ringsystem H | $C_2H_5$ | 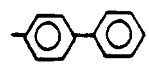 |
| 34 | Ringsystem H | $C_2H_5$ | 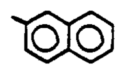 |
| 35 | Ringsystem H | H | $-CH_2-$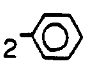 |
| 36 | Ringsystem H | $C_2H_5$ | $-CH(C_6H_5)_2$ |
| 37 | Ringsystem H | $C_2H_5$ | $-(CH_2)_8-$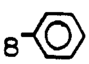 |
| 38 | Ringsystem I | $CH_3$ | 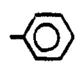 |
| 39 | Ringsystem J | $C_2H_5$ | $-CH(C_6H_5)_2$ |
| 40 | Ringsystem K | $C_2H_5$ | $-(CH_2)_4-CH_3$ |
| 41 | Ringsystem L | $C_2H_5$ | $-C(CH_3)_3$ |

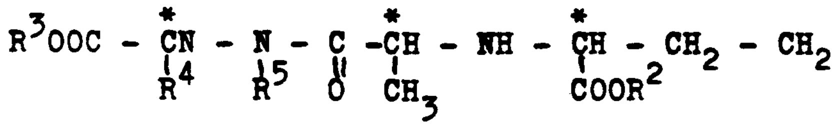

| Bsp. | 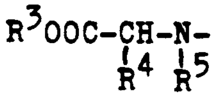 | $R^2$ | $R^3$ |
|---|---|---|---|
| 42 | Ringsystem M | $C_2H_5$ | $-(CH_2)_2-CH(CH_3)_3$ |
| 44 | Ringsystem N | $C_2H_5$ | $-CH(CH_3)_2$ |
| 45 | Ringsystem O | $C_2H_5$ | $-(CH_2)_2-$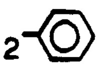 |
| 46 | Ringsystem P | $C_2H_5$ | 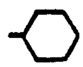 |
| 48 | Ringsystem A | $CH_2CH(CH_3)_2$ | $-CH_2-$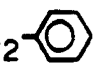 |
| 49 | Ringsystem B | $CH(CH_3)_2$ | $-C(CH_3)_3$ |
| 50 | Ringsystem C | $CH(C_6H_5)_2$ | -H |
| 51 | Ringsystem D | $CH_2-$ | $-CH_2-$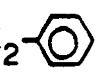 |
| 52 | Ringsystem D | $CH_2-CH(CH_3)_2$ | $-CH_2-$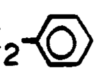 |
| 53 | Ringsystem D | $CH_2-CH(CH_3)_2$ | -H |
| 54 | Ringsystem D | $CH_2-CH(CH_3)_2$ | $-CH(C_6H_5)_2$ |
| 55 | Ringsystem D | $CH(C_6H_5)_2$ | $-C(CH_3)_3$ |
| 56 | Ringsystem D | $CH(C_6H_5)_2$ | -H |

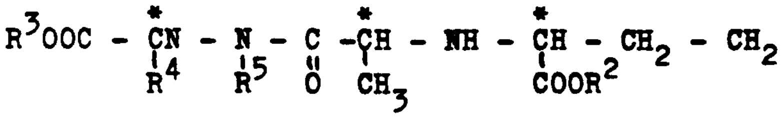

| Bsp. | 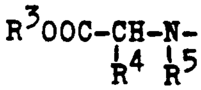 | $R^2$ | $R^3$ |
|---|---|---|---|
| 57 | Ringsystem E | 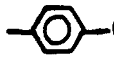 $-CH_3$ | $-(CH_2)_5-CH_3$ |
| 59 | Ringsystem G | $-CH(C_6H_5)_2$ | $-CH_2-$ 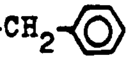 |
| 60 | Ringsystem G | $-CH(C_6H_5)_2$ | $-C(CH_3)_3$ |
| 61 | Ringsystem H | $-CH_2CH(CH_3)_2$ | $-CH_2-$ 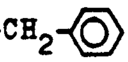 |
| 62 | Ringsystem H | $-CH_2CH(CH_3)_2$ | -H |
| 63 | Ringsystem H | $-CH_2CH(CH_3)_2$ | $-CH(C_6H_5)_2$ |
| 64 | Ringsystem H | $-CH_2-$ 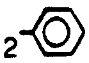 | $-CH_2-$ 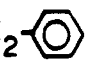 |
| 65 | Ringsystem H | $-CH_2-$ 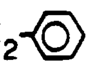 | $-C(CH_3)_3$ |
| 66 | Ringsystem H | $-CH(C_6H_5)_2$ | $-CH_2-$ 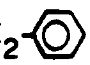 |
| 67 | Ringsystem H | $-CH(C_6H_5)_2$ | -H |
| 68 | Ringsystem H | $-CH(C_6H_5)_2$ | $-C(CH_3)_3$ |
| 69 | Ringsystem H | $-CH(C_6H_5)_2$ | $-CH(C_6H_5)_2$ |
| 70 | Ringsystem I | 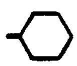 | 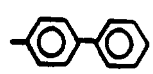 |
| 71 | Ringsystem J | $-(CH_2)_5-CH_3$ | $-CH(C_6H_5)_2$ |

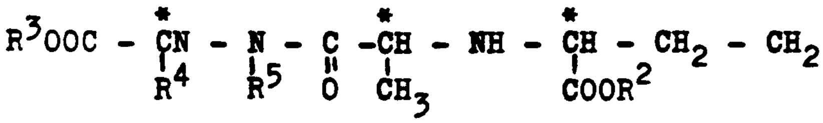

| Bsp. | 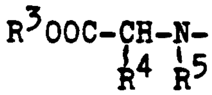 | $R^2$ | $R^3$ |
| --- | --- | --- | --- |
| 72 | Ringsystem K | 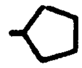 | $-CH_2-$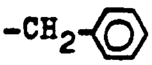 |
| 73 | Ringsystem L | $-CH_2)_2-$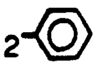 | $-(CH_2)_5-CH_3$ |
| 74 | Ringsystem M | $-(CH_2)_2-CH(CH_3)_2$ | $-CH(C_6H_5)_2$ |
| 75 | Ringsystem N | $-CH(C_6H_5)_2$ | $-C(CH_3)_2-CH_2CH_3$ |
| 76 | Ringsystem O | $-CH(CH_3)_2$ | $-CH(C_6H_5)_2$ |
| 77 | Ringsystem P | $-CH(CH_3)_2$ | $-C(CH_3)_3$ |
| 78 | Ringsystem Q | $-C(CH_3)_3$ | $-CH_2-$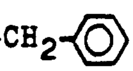 |
| 78a | Ringsystem D | $C_2H_5$ | Menthyl |
| 78b | Ringsystem G | Menthyl | $CH_2C_6H_5$ |
| 83 | 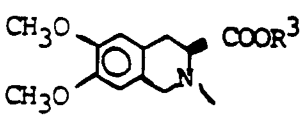 | $-CH(C_6H_5)_2$ | $-NH-CH-(CH_2)_2-$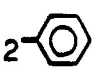 |

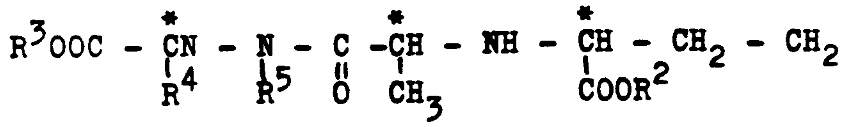

| Bsp. | 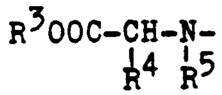 | $R^2$ | $R^3$ |
|---|---|---|---|
| 85 | $R^3OOC-CH_2-N-$ | 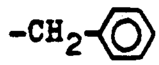 | 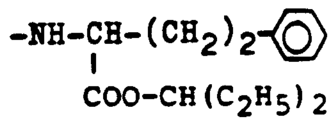 |

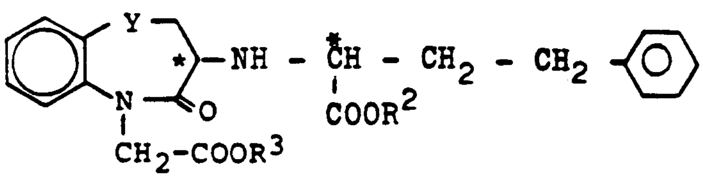

| Bsp. | $Y^1$ | $R^2$ | $R^3$ |
|---|---|---|---|
| 87 | $CH_2$ | $-C_2H_5$ | $-CH(C_6H_5)_2$ |
| 89 | $CH_2$ | $-CH_2-CH(CH_3)_2$ | 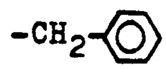 |

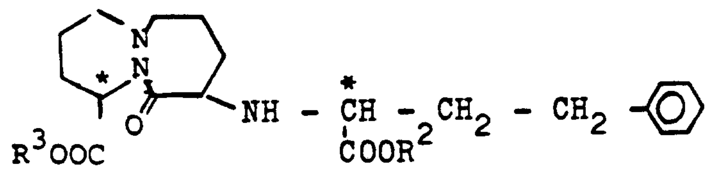

| Bsp. | $R^2$ | $R^3$ |
|---|---|---|
| 90 | $-CH(C_6H_5)_2$ | $-C(CH_3)_3$ |
| 91 | $-CH(C_6H_5)_2$ | $-CH(C_6H_5)_2$ |

Beispiel 92

4-[N-(1S)-Carboethoxy-3-phenylpropyl)-S-benzyl]-exospiro(bicyclo[2.2.2]octan-2,3-pyrrolidin)-5-carbonsäure

a) N-(1S-Carbethoxy-3-phenylpropyl)-S-laurylbenzylester

3,4 g (10 mmol) 2-(D)-Trifluormethylsulfonyloxy-4-phenylbuttersäureethylester und 5,9 g (15 mmol) L-Leucin benzylestertosylat wurden in 50 ml abs. $CH_2Cl_2$ gemischt und nach Zusatz von 4,2 ml Triethylamin 6,5 Stunden bei Raumtemperatur gerührt. Nach Einengen der Lösung wurde das Produkt durch Säulenchromatographie (Kieselgel, Cyclohexan/Essigester 9:1) isoliert. Es wurden 3,2 g farbloses Öl erhalten.

$^1$H-NMR $\delta$ = 0,95 (d, $CH_3$), 1,2 (t, $CH_3$) 1,8 (m, $CH_2$) 2,6 (m, $CH_2$) 3,3 (m, CH) 4,1 (q, $CH_2$) 5,1 (s, $CH_2$) 7,3 (s, CH) ppm.

b) N-(1-S-Carboethoxy-3-phenylpropyl)-S-leucin

3,1 g des in a) erhaltenen Benzylesters wurden mit 500 mg Pt/C (10 %) in 200 ml Ethanol hydrogenolytisch gespalten. Nach Filtration von Katalysator und Einengen der Lösung wurden 2,3 g farbloses Kristallisat der Carbonsäure vom Schmelzpunkt 120-121 °C erhalten.

$^1$H-NMR $\delta$ = 0,9 (d, $CH_3$), 1,25 (t, $CH_3$), 1,8-2,1 (m,$CH_2$), 2,7 (m, $CH_2$), 3,3 (q, CH), 4,25 (q, $CH_2$), 7,2 (s, CH) ppm.

c) 4-[N-(1S)-Carbethoxy-3-phenylpropyl)-S-leucyl]-exo-spirobicyclo[2.2.2]octan-2,3-pyrrolidin-5-carbonsäure

2 g (6,2 mmol) N-(1-S-Carboethoxy-3-phenylpropyl)-S-leucin und 1,9 g (3,9 mmol) exo-Spiro(bicyclo-[2.2.2]-octan-2,3-pyrrolidin)-5-carbonsäurebenzylester wurden in 100 ml Dimethylformamid mit 4,3 ml Triethylamin und 6,5 ml n-Propylphosphonsäureanhydrid über Nacht bei Raumtemperatur gerührt. Die Reaktionslösung wurde in Essigester aufgenommen und zweimal gegen wäßrige $NaHCO_3$-Lösung, sowie je einmal gegen wäßrige 10 %-Zitronensäure, wäßrige ges. $NaHCO_3$-Lösung und ges. wäßrige NaCl-Lösung geschüttelt. Die organische Phase wurde danach abgetrennt, getrocknet und eingeengt. Das Rohprodukt mit einer Ausbeute von 2,8 g wurde mittels Säulenchromatographie (Kieselgel, Toluol/Essigester 95:5) in die beiden Diastereomeren getrennt. Von jedem Benzylester wurden 1g reines Produkt erhalten. 1 g des ersten Diastereomers wurden mit Pd/C in 40 ml Ethanol hydriert. Es wurden 780 mg kristalline Carbonsäure vom Schmelzpunkt 131-132 °C erhalten.

Drehwert $[\alpha]_D^{20}$ = -2,8 ° (c = 1, Methanol)

860 mg des zweiten Diastereomeren wurden mit Pd/C in 35 ml Ethanol hydriert und die Ausbeute nach Abfiltration vom Katalysator und Einengen der Lösung betrug 720 mg. Schmelzpunkt: Die Substanz sintert ab 65 °C

Drehwert $[\alpha]_D^{20}$ = -22,2 ° (c = 1, Methanol)

Beispiel 93

4-[N-(1S)-Carboxyl-3-phenylpropyl)-S-leucyl]-exo-spiro(bicyclo[2.2.2]octan-2,3-pyrrolidin)-5-carbonsäure

102 mg (0,2 mmol) der Carbonsäure aus Beispiel 92c) wurden in wäßriger 4N KOH-Lösung angerührt bis sich die gesamte Substanz aufgelöst hatte. Die Lösung wurde auf einen Ionenaustauscher ((R)Amberlite R 120) gegeben und mit wäßriger 2 %iger Pyridin-Lösung eluiert. Die Ausbeute betrug 70 mg.
Drehwert $[\alpha]_D^{20}$ = +3,9° (c= 1, Methanol)
Die folgenden Verbindungen der Formel II werden in analoger Weise hergestellt (die Bezeichnungen der Ringsysteme entspricht derjenigen für die Verbindungen der allgemeinen Formeln I und II):

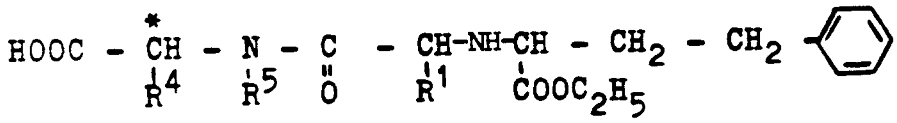

| $R^1$ | 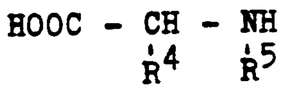 |
|---|---|
| $CH(CH_3)_2$ | A |
| $CH(CH_3)CH_2CH_3$ | A |
| $CH_2C_6H_5$ | A |
| $CH_2-C_6H_{11}$ ($CH_2$-Cyclohexyl) | C |
| $CH_2-C_6H_4$ $OCH_3$ | G |
| $CH_2-C_6H_4$ $OC_2H_5$ | C |
| $CH_2-C_6H_4-OC_3H_7$ | C |
| $CH_2-C_6H_4-OC_4H_9$ | A |
| $(CH_2)_4-NH_2$ | D |
| $(CH_2)_3-NH_2$ | A |
| $CH_2-CH_2-S$ $CH_3$ | A |

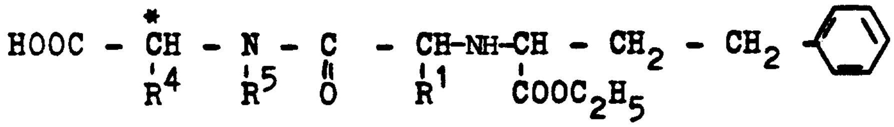

| $R^1$ | 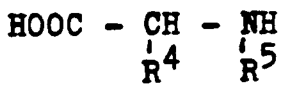 |
| --- | --- |
| $CH(CH_3)_2$ | B |
| $CH(CH_3)CH_2CH_3$ | B |
| $CH_2C_6H_5$ | B |
| $CH_2-C_6H_{11}$ | D |
| $CH_2-C_6H_4-OCH_3$ | H |
| $CH_2-C_6H_4-OC_2H_5$ | G |
| $CH_2-C_6H_4-On-C_3H_7$ | G |
| $CH_2-C_6H_4-On-C_4H_9$ | C |
| $(CH_2)_4-NH_2$ | C |
| $(CH_2)_3-NH_2$ | G |
| $CH_2-CH_2-S-CH_3$ | D |

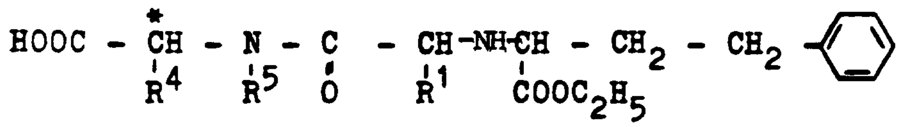

| $R^1$ | 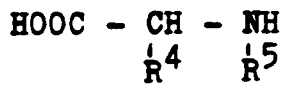 |
| --- | --- |
| $CH(CH_3)_2$ | C |
| $CH(CH_3)CH_2CH_3$ | C |
| $CH_2C_6H_5$ | C |
| $CH_2-C_6H_{11}$ | E |
| $CH_2-C_6H_4-OCH_3$ | I |
| $CH_2-C_6H_4-OC_2H_5$ | H |
| $CH_2-C_6H_4-On-C_3H_7$ | H |
| $CH_2-C_6H_4-On-C_4H_9$ | G |
| $(CH_2)_4-NH_2$ | G |
| $(CH_2)_3-NH_2$ | H |
| $CH_2-CH_2-S-CH_3$ | H |

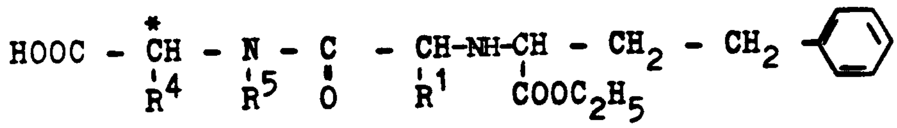

| $R^1$ | HOOC - CH - NH <br> $R^4$ $R^5$ |
|---|---|
| $CH(CH_3)_2$ | D |
| $CH(CH_3)CH_2CH_3$ | D |
| $CH_2C_6H_5$ | D |
| $CH_2-C_6H_{11}$ | F |
| $CH_2-C_6H_4-OCH_3$ | N |
| $CH_2-C_6H_4-OC_2H_5$ | I |
| $CH_2-C_6H_4-On-C_3H_7$ | I |
| $CH_2-C_6H_4-On-C_4H_9$ | H |
| $(CH_2)_4-NH_2$ | H |
| $(CH_2)_3-NH_2$ | O |
| $CH_2-CH_2-S-CH_3$ | I |

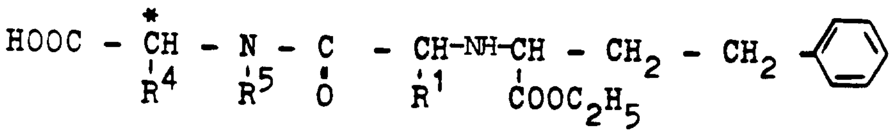

| $R^1$ | 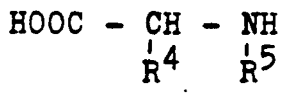 |
|---|---|
| $CH(CH_3)_2$ | G |
| $CH(CH_3)CH_2CH_3$ | E |
| $CH_2C_6H_5$ | H |
| $CH_2-C_6H_{11}$ | G |
| $CH_2-C_6H_4-OCH_3$ | O |
| $CH_2-C_6H_4-OC_2H_5$ | N |
| $CH_2-C_6H_4-On-C_3H_7$ | N |
| $CH_2-C_6H_4-On-C_4H_9$ | I |
| $(CH_2)_4-NH_2$ | N |
| $(CH_2)_3-NH_2$ | P |
| $CH_2-CH_2-S-CH_3$ | P |

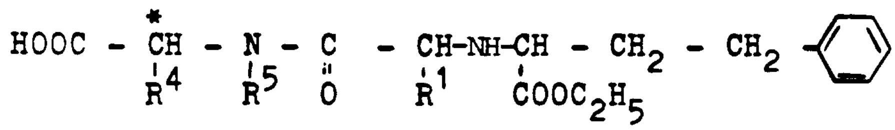

| $R^1$ | 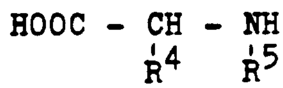 |
|---|---|
| $CH(CH_3)_2$ | H |
| $CH(CH_3)CH_2CH_3$ | F |
| $CH_2C_6H_5$ | I |
| $CH_2-C_6H_{11}$ | H |
| $CH_2-C_6H_4-OCH_3$ | P |
| $CH_2-C_6H_4-OC_2H_5$ | O |
| $CH_2-C_6H_4-On-C_3H_7$ | O |
| $CH_2-C_6H_4-On-C_4H_9$ | N |
| $(CH_2)_4-NH_2$ | Q |
| $(CH_2)_3-NH_2$ | Q |
| $CH_2-CH_2-S\ CH_3$ | Q |
| 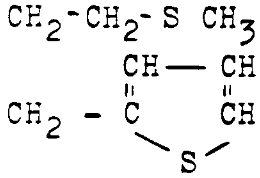 | G |

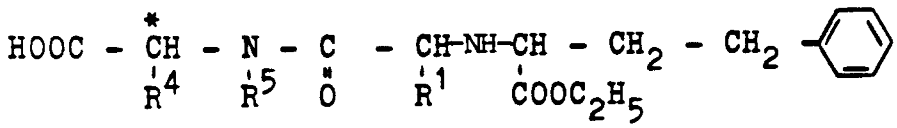

| $R^1$ | $HOOC-\underset{R^4}{CH}-\underset{R^5}{NH}$ |
|---|---|
| $CH(CH_3)_2$ | I |
| $CH(CH_3)CH_2CH_3$ | G |
| $CH_2C_6H_5$ | N |
| $CH_2-C_6H_{11}$ | I |
| $CH_2-C_6H_4-OCH_3$ | Q |
| $CH_2-C_6H_4-OC_2H_5$ | P |
| $CH_2-C_6H_4-On-C_3H_7$ | P |
| $CH_2-C_6H_4-On-C_4H_9$ | O |
| $(CH_2)_4-NH_2$ | P |
| $CH_2-CH_2-S-CH_3$ | N |

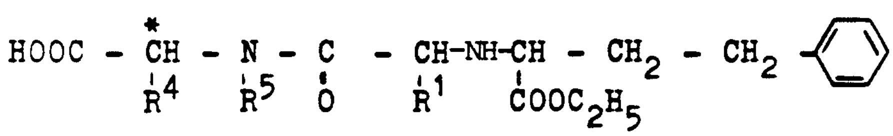

| $R^1$ | $HOOC-\underset{R^4}{CH}-\underset{R^5}{NH}$ |
|---|---|
| $CH(CH_3)_2$ | O |
| $CH(CH_3)CH_2CH_3$ | H |
| $CH_2C_6H_5$ | O |
| $CH_2-C_6H_{11}$ | L |
| $CH_2-C_6H_4-OC_2H_5$ | Q |
| $CH_2-C_6H_4-On-C_3H_7$ | Q |
| $CH_2-C_6H_4-On-C_4H_9$ | P |
| $CH_2-CH_2-S-CH_3$ | G |

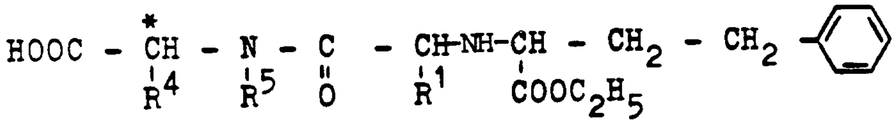

| $R^1$ | 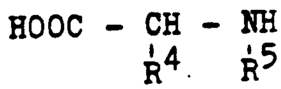 |
|---|---|
| $CH(CH_3)_2$ | P |
| $CH_2-C_6H_5$ | P |
| $CH_2-C_6H_{11}$ | M |
| $CH_2-C_6H_4-OCH_3$ | D |
| $CH_2-C_6H_4-OC_2H_5$ | D |
| $CH_2-C_6H_4-On-C_3H_7$ | D |
| $CH_2-C_6H_4-On-C_4H_9$ | Q |

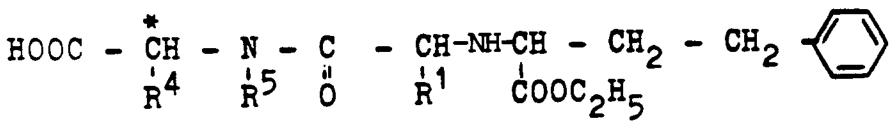

| $R^1$ | 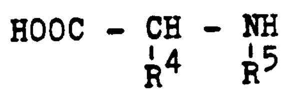 |
|---|---|
| $CH(CH_3)_2$ | N |
| $CH(CH_3)CH_2CH_3$ | P |
| $CH_2-C_6H_5$ | Q |
| $CH_2-C_6H_{11}$ | N |
| $CH_2-C_6H_4-On-C_4H_9$ | D |
| $CH(CH_3)CH_2CH_3$ | Q |
| $CH(CH_3)CH_3CH_3$ | I |
| $CH_2-C_6H_{11}$ | O |
| $CH_2-C_6H_{11}$ | Q |

Beispiel 94:

1-[N-(1S)-Carboethoxybutyl)-S-alanyl]-octahydrocyclopenta[b]pyrrol)-2-carbonsäure

a) DL-2-Trifluormethylsulfonyloxy-pentansäureethylester
5 g (34 mmol) 2-Hydroxyvaleriansäureethylester und 2,85 g (35,9 mmol) absl. Pyridin wurden in 100 ml abs. $CH_2Cl_2$ unter Schutzgas gelöst, auf 0 °C gekühlt und mit 9, 66 g (34 mmol) Trifluormethansulfonsäureanhydrid versetzt. Nach Aufwärmen auf Raumtemperatur wurde 6 Stunden gerührt. Nach Einengen der Lösung wurde das erhaltene Rohprodukt säulenchromatographisch gereinigt (Kieselgel, Petrolether/$CH_2Cl_2$ 6:1). Die Ausbeute betrug 9,3 g einer farblosen leicht viskosen Flüssigkeit.

| IR: | 2880 - 3000<br>1200 - 1220 | 1770, 1420,<br>1150, 620 $cm^{-1}$ |
|---|---|---|

b) N-(1-S-Carboethoxybutyl)-S-alaninbenzylester
4,9 g (17,6 mmol) des so erhaltenen Trifluormethansulfonsäureesters wurden unter Stickstoff in 70 ml $CH_2Cl_2$ abs. mit 4,08 g L-Alaninbenzylester-hydrochlorid(19 mmol) unter Zusatz von 5,4 ml Triethylamin gelöst und 3 Stunden bei Raumtemperatur gerührt. Die Lösung wurde eingeengt, das Rohprodukt in Essigester aufgenommen und dreimal mit Wasser gewaschen, getrocknet und eingeengt. Die Diastereomeren wurde durch eine Säulenchromatographie (Kieselgel, Cyclohexan/Essigester (5:1) getrennt. Die Ausbeute betrug pro Isomer 500 mg. Das in der ersten Filtration isolierte Diastereomer hat die S,S-Konfiguration.
$^1$H-NMR
δ = 0,9 (t,$CH_3$), 1,3 (t,$CH_3$), 1,35 (d,$CH_3$), 1,4 (m,$CH_2$), 1,6 (m,$CH_2$), 1,9 (s,NH), 3,3 (t,CH), 3,4 (q,CH), 4,2 (m,$CH_2$), 5,15 (q,$CH_2$ Ph), 7,4 (s, CH aromat.) ppm.

c) N-(1-S-Carboethoxybutyl)-S-alanin
600 mg (1,95 mmol) Benzylester (Diastereomer A) wurden in 34 ml Ethanol mit Pd auf Kohle hydriert. Der Katalysator wurde anschließend abfiltriert und die Lösung in Vakuum eingeengt. Das Produkt fiel danach als weißer Feststoff mit einem Schmelzpunkt von 137° und einer Ausbeute von 430 mg an.

d) 430 mg (1,98 mmol) N-(1-S-Carboethoxybutyl)-S-alanin und 486 mg (1,98 mmol) L(-)-Octahydrocyclopenta-[b]-pyrrol-2-carbonsäurebenzylester wurden unter Stickstoff in 20 ml Dimethylformamid gelöst, auf -10 °C gekühlt, mit 1,5 ml Triethylamin und 2 ml n-Propylphosphonsäureanhydrid versetzt. Es wurde 1 Stunde bei -10 °C und danach über Nacht bei Raumtemperatur gerührt. Die Lösung wurde in 200 ml Essigester aufgenommen und mit ges. wäßriger $NaHCO_3$-Lösung 10 % wäßriger Citronensäure und ges. wäßriger NaCl-Lösung gewaschen. Nach Trocknen und Einengen der Lösung wurde die diastereomeren Verbindungen durch Säulenchromatographie (Kieselgel, Cyclohexan/Essigester 9:1) getrennt. Die Ausbeute betrug 360 mg. Beide Diastereomere werden wie in Beispiel 94 c) beschrieben mit Pd/C in Ethanol hydriert und fielen nach Einengen der Lösung als weißer Feststoff an.

Beispiel 95:

1-[N-(1S)-Carboxylbutyl)-S-alanyl]-(octahydrocylopenta[b]pyrrol)-2-carbonsäure

60 mg (0,17 mmol) Carbonsäure (Beispiel 94 d) wurden in 2 ml 4N wäßriger KOH-Lösung eingerührt, bis zur vollständigen Auflösung der Substanz. Danach wurde die Lösung auf einen stark sauren Ionenaustauscher gegeben und mit wäßriger 2 %iger Pyridin-Lösung eluiert. Nach Einengen der Lösung betrug die Ausbeute 39 mg.

Analog der in den Beispielen 94 und 95 hergestellten Verbindungen lassen sich gemäß der Formel II

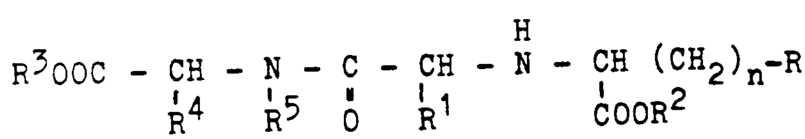

worin mit n = 2
$R^1 = CH_3$
$R^2 = C_2H_5$
$R^3 = H$
sowie R und der Molekülteil

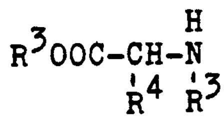

entsprechend der nachstehend aufgeführten Tabelle substituiert sind, folgende weitere Verbindungen synthetisieren.

| R | 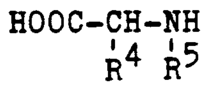 |
| --- | --- |
| $CH_3$ | A |
| $-CH_2CH_2CH_3$ | A |
| $-(CH_2)_9-CH_3$ | C |
| 2-Naphthyl | D |
| 4-Biphenylyl | A |
| $-(CH_2)_{13}-CH_3$ | A |

| R | 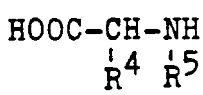 |
| --- | --- |
| $CH_3$ | B |
| $-CH_2CH_2CH_3$ | B |
| $-(CH_2)_9-CH_3$ | D |
| 2-Naphthyl | F |
| 4-Biphenylyl | C |
| $-(CH_2)_{13}-CH_3$ | B |

| R | HOOC-CH(R^4)-NH(R^5) |
|---|---|
| $CH_3$ | C |
| $-CH_2CH_2CH_3$ | C |
| $-(CH_2)_9-CH_3$ | G |
| 2-Naphthyl | G |
| 4-Biphenylyl | D |
| $-(CH_2)_{13}-CH_3$ | C |

| R | HOOC-CH(R^4)-NH(R^5) |
|---|---|
| $CH_3$ | D |
| $-CH_2CH_2CH_3$ | D |
| $-(CH_2)_9-CH_3$ | H |
| 2-Naphthyl | H |
| 4-Biphenylyl | H |
| $-(CH_2)_{13}-CH_3$ | D |

| R | HOOC-CH(R^4)-NH(R^5) |
|---|---|
| $CH_3$ | G |
| $-CH_2CH_2CH_3$ | G |
| $-(CH_2)_9-CH_3$ | K |
| 2-Naphthyl | I |
| 4-Biphenylyl | L |
| $-(CH_2)_{13}-CH_3$ | G |

| R | $HOOC-CH(R^4)-NH(R^5)$ |
|---|---|
| $CH_3$ | H |
| $-CH_2CH_2CH_3$ | H |
| $-(CH_2)_9-CH_3$ | N |
| 2-Naphthyl | M |
| 4-Biphenylyl | N |
| $-(CH_2)_{13}-CH_3$ | N |

| R | $HOOC-CH(R^4)-NH(R^5)$ |
|---|---|
| $CH_3$ | N |
| $-CH_2CH_2CH_3$ | N |
| $-(CH_2)_9-CH_3$ | O |
| 2-Naphthyl | N |
| 4-Biphenylyl | P |
| $-(CH_2)_{13}-CH_3$ | O |

| R | $HOOC-CH(R^4)-NH(R^5)$ |
|---|---|
| $CH_3$ | Q |
| $-CH_2CH_2CH_3$ | Q |
| $-(CH_2)_9-CH_3$ | P |
| 4-Biphenylyl | Q |
| $-(CH_2)_{13}-CH_3$ | P |

| R | HOOC-CH-NH $R^4$ $R^5$ |
|---|---|
| $CH_3$ | P |
| $-CH_2CH_2CH_3$ | P |
| $-(CH_2)_9-CH_3$ | Q |
| 4-Biphenylyl | G |
| $-(CH_2)_{13}-CH_3$ | Q |
| 4-Biphenylyl | H |

**Patentansprüche**

1. Verwendung von ACE-Inhibitoren der Formel (II) oder deren physiologisch verträglichen Salz bei der Herstellung eines Arzneimittels mit nootroper Wirkung, wobei in Formel (II)

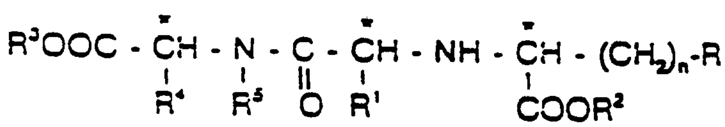

(II)

bedeuten:

| | |
|---|---|
| n = | 1 oder 2 |
| R = | Wasserstoff;<br>einen aliphatischen Rest mit 1 bis 21 C-Atomen;<br>einen aromatischen Rest mit 6 bis 12 C-Atomen; |
| $R^1$ = | Wasserstoff;<br>einen aliphatischen Rest mit 1 bis 21 C-Atomen oder,<br>falls von vorstehenden Definitionen nicht schon umfaßt,<br>die erforderlichenfalls geschützte Seitenkette einer natürlich verkommenden α-Aminosäure bedeutet, |
| $R^2$ und $R^3$ | gleich oder verschieden sind und Wasserstoff;<br>einen aliphatischen Rest mit 1 bis 21 C-Atomen;<br>einen alicyclischen Rest mit 3 bis 20 C-Atomen;<br>einen aromatischen Rest mit 6 bis 12 C-Atomen;<br>einen araliphatischen Rest mit 7 bis 32 C-Atomen bedeuten und |
| $R^4$ und $R^5$ | zusammen mit den sie tragenden Atomen ein mono-, bi- oder tricyclisches Ringsystem aus der Reihe Pyrrolidin, Tetrahydroisochinolin, Decahydroisochinolin, Octahydroindol, Indolin, Octahydrocyclopenta[b]pyrrol, 2-Azaspiro[4.5]decan, 2-Azaspiro-[4.4]nonan, Spiro[(bicyclo[2.2.1]heptan)-2,3'-pyrrolidin],Spiro[(bicyclo-[2.2.2]octan)-2,3'-pyrrolidin], 2-Azatricyclo[4.3.0.$1^{6,9}$]decan, Decahydrocyclohepta[b]pyrrol, Octahydroisoindol, Octahydrocyclopenta[c]pyrrol, 2,3,3a,4,5,6a-Hexahydroindol, 1,2,-3,3a,4,5a-Hexahydrocyclopenta[b]pyrrol und 2-Azabicyclo[3.1.0]hexan bilden. |

2. Verwendung gemäß Anspruch 1, dadurch gekennzeichnet, daß (S,S,S,S,S)-1-[N-(1-Carbethoxy-3-phenyl-propyl)-alanyl]-octahydoindol-2-carbonsäure, 1-[N-(1-S-Carbethoxy-3-phenylpropyl)-S-alanyl]-(2S,3aR,7aS)-octahydroindol-2-carbonsäure, (S,S,S,S,S)-2-[N-(1-Carbethoxy-3- phenylpropyl)-alanyl]-decahydroisochinolin-3-carbonsäure, (S,S,S)-2-[N-(1-Carbethoxy-3-phenylpropyl)-alanyl]-tetrahydroisochinolin-3-carbonsäure, (S,S,S,S,S)-2-[N-(1-Carbethoxy-3-phenylpropyl)-alanyl]-2-azabicyclo[3.3.0]octan-3-carbonsäure, 2-N-(1-S-Carbethoxy-3-phenylpropyl)-S-alanyl]-cis,endo-2-azabicyclo[3.1.0]hexan-3-S-carbonsäure oder 1-[N-(1-S-Carbethoxy-3-phenylpropyl)-S-alanyl]-cis,endo-1H-2,3,3a,4,5,7a-hexahydroindol-2-S- oder -2-R-carbonsäure verwendet wird.

3. Verwendung gemäß Anspruch 1, dadurch gekennzeichnet, daß 1'-[N-(1-S-Carbethoxy-3-phenyl-propyl)-S-alanyl]-exo/endo-spirobicyclo[2.2.2]octan-2,3'pyrrolidin-5'-carbonsäure, 1'-[N-(1-S-Carbethoxy-3-phenyl-propyl)-S-alanyl]-(3'S,5'S)-spiro-bicyclo[2.2.2]-octan-2,3'-pyrrolidin-5'-carbonsäure, 1'[N-(1-S-Carbethoxy-3-phenylpropyl)-S-alanyl]-(3'S,5'R)-spiro-bicyclo[2.2.2]octan-2,3'-pyrrolidin-5'-carbon säure, 1'-[N-(1-S-Carbethoxy-3-phenylpropyl)-S-alanyl]-(3'R,5'S)-spiro-bicyclo[2.2.2]octan-2,3'-pyrrolidin-5'-carbonsäure 1'-[N-(1-S-Carbethoxy-3-phenylpropyl)-S-alanyl]-(3'R,5'R)-spiro-bicyclo[2.2.2]octan-2,3'-pyrrolidin-5'-carbonsäure, 1'-[N-(1-R-Carbethoxy-3-phenylpropyl)-S-alanyl]-(3'S,5'S)-spiro-bicyclo[2.2.2]-octan-2,3'-pyrrolidin-5'-carbonsäure, 1'-[N-(1-R-Carbethoxy-3-phenylpropyl)-S-alanyl]-(3'S,5'R)-spiro-bicyclo[2.2.2]octan-2,3'-pyrrolidin-5'-carbonsäure, 1'-[N-(1-R-Carbethoxy-3-phenylpropyl)-S-alanyl]-(3'R,5'S)-spiro-bicyclo[2.2.2]octan-2,3'-pyrrolidin-5'-carbonsäure, 1'-[N (1-R-Carbethoxy-3-phenylpropyl)-S-alanyl]-(3'R,5'R)-spiro-bicyclo[2.2.2]octan-2,3'-pyrrolidin-5'-carbonsäure, 1'-[N-(1-S-Carbethoxy-3-phenylpropyl)-R-alanyl]-(3'S, 5'S)-spiro-bicyclo[2.2.2]octan-2,3'-pyrrolidin-5'-carbonsäure, 1'-[N-(1-S-Carbethoxy-3-phenylpropyl)-R-alanyl]-(3'R,5'S)-spiro-bicyclo[2.2.2]octan-2,3'-pyrrolidin-5'-carbonsäure, 1'-[N-(1-S-Carbethoxy-3-phenylpropyl)-R-alanyl]-(3'S,5'R)-spiro-bicyclo[2.2.2]octan-2,3'-pyrrolidin-5'-carbonsäure, 1'-[N-(1-S-Carbethoxy-3-phenylpropyl)-R-alanyl]-(3'R,5'R)-spiro-bicyclo[2.2.2]octan-2,3'-pyrrolidin-5'-carbonsäure, 1'-[N-(1-R-Carbethoxy-3-phenypropyl)-R-alanyl]-(3'S,5'S)-spiro-bicyclo[2.2.2]-octan-2,3'-pyrrolidin-5'-carbonsäure, 1'-[N-(1-R-Carbethoxy-3-phenylpropyl)-R-alanyl]-(3'R,5'S)-spiro-bicyclo[2.2.2]-octan-2,3'-pyrrolidin-5'-carbonsäure 1'-[N-(1-R-Carbethoxy-3-phenylpropyl)-R-alanyl]-(3'S, 5'R)-spiro-bicyclo[2.2.2]octan-2,3'-pyrrolidin-5'-carbonsäure oder 1'-[N-(1-R-Carbethoxy-3-phenylpropyl)-R-alanyl]-(3'R,5'R)-spiro-bicyclo[2.2.2]octan-2,3'-pyrrolidin-5'-carbonsäure verwendet wird.

## Claims

1. The use of ACE inhibitors of the formula (II) or their physiologically tolerated salt for the preparation of a medicament having a nootropic action, where in formula (II)

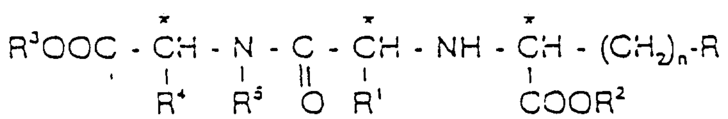

(II)

| | |
|---|---|
| n | is 1 or 2; |
| R | denotes hydrogen,<br>an aliphatic radical having 1-21 carbon atoms,<br>an aromatic radical having 6-12 carbon atoms, |
| $R^1$ | denotes hydrogen,<br>an aliphatic radical having 1-21 carbon atoms, or if not already covered by the above definitions, the side-chain, protected where necessary, of a naturally occurring α-amino acid, |
| $R^2$ and $R^3$ | are identical or different and denote hydrogen,<br>an aliphatic radical having 1-21 carbon atoms,<br>an alicyclic radical having 3-20 carbon atoms,<br>an aromatic radical having 6-12 carbon atoms,<br>an araliphatic radical having 7-32 carbon atoms, and |
| $R^4$ and $R^5$ | form, together with the atoms carrying them, a mono-, bi- or tricyclic ring system from the series comprising pyrrolidine, tetrahydroisoquinoline, decahydroisoquinoline, octahydroindole, indoline, octahydrocyclopenta[b]pyrrole, 2-azaspiro[4,5]decane, 2-azaspiro[4,4]nonane, spiro[(bicyclo[2.2.1]heptane)-2,3'-pyrrolidine], spiro[(bicyclo-[2.2.2]octane)-2,3-pyrrolidine], 2-azatricyclo[4.3.0.$1^{6,9}$]decane, decahydrocyclohepta-[b]pyrrole, octahydroisoindole, octahydrocyclopenta[c]pyrrole, 2,3,3a,4,5,6a-hexahydroindole, 1,2,3,3a,4,5a-hexahydrocyclopenta[b]pyrrole and 2-azabicyclo[3.1.0]-hexane. |

2. The use as claimed in claim 1, wherein use is made of (S,S,S,S,S)-1-[N-(1-carbethoxy-3-phenylpropyl)-alanyl]-octahydroindole-2-carboxylic acid, 1-[N-(1-S-carbethoxy-3-phenylpropyl)-S-alanyl]-(2S,3aR,7aS)-octahydroindole-2-carboxylic acid, (S,S,S,S,S)-2-[N-(1-carbethoxy-3-phenylpropyl)-alanyl]-decahydroisoquinoline-3-carboxylic acid, (S,S,S)-2-[N-(1-carbethoxy-3-phenylpropyl)-alanyl]-tetrahydroisoquinoline-3-carboxylic acid, (S,S,S,S,S)-2-[N-(1-carbethoxy-3-phenylpropyl)-alanyl]-2-azabicyclo[3.3.0]octane-3-carboxylic acid, 2-N-(1-S-carbethoxy-3-phenylpropyl)-S-alanyl]-cis,endo-2-azabicyclo[3.1.0]hexane-3-S-carboxylic acid or 1-[N-(1-S-carbethoxy-3-phenylpropyl)-S-alanyl]-cis,endo-1H-2,3,3a,4,5,7a-hexahydroindole-2-S- or -2-R-carboxylic acid.

3. The use as claimed in claim 1, wherein use is made of 1'-[N-(1-S-carbethoxy-3-phenylpropyl)-S-alanyl]-exo/endo-spirobicyclo[2.2.2]octane-2,3'-pyrrolidine-5'-carboxylic acid, 1'-[N-(1-S-carbethoxy-3-phenylpropyl)-S-alanyl]-(3'S,5'S)-spirobicyclo[2.2.2]octane-2,3'-pyrrolidine-5'-carboxylic acid, 1'-[N-(1-S-carbethoxy-3-phenylpropyl)-S-alanyl]-(3'S,5'R)-spirobicyclo[2.2.2]-octane-2,3'-pyrrolidine-5'-carboxylic acid, 1'-[N-(1-S-carbethoxy-3-phenylpropyl)-S-alanyl]-(3'R,5'S)-spirobicyclo[2.2.2]octane-2,3'-pyrrolidine-5'-carboxylic acid, 1'-[N-(1-S-carbethoxy-3-phenylpropyl)-S-alanyl]-(3'R,5'R)-spirobicyclo[2.2.2]octane-2,3'-pyrrolidine-5'-carboxylic acid, 1'-[N-(1-R-carbethoxy-3-phenylpropyl)-S-alanyl]-(3'S,5'S)-spirobicyclo-[2.2.2]octane-2,3'-pyrrolidine-5'-carboxylic acid, 1'-[N-(1-R-carbethoxy-3-phenylpropyl)-S-alanyl]-(3'S,5'R)-spirobicyclo[2.2.2]octane-2,3'-pyrrolidine-5'-carboxylic acid, 1'-[N-(1-R-carbethoxy-3-phenylpropyl)-S-alanyl]-(3'R,5'S)-spirobicyclo[2.2.2]octane-2,3'-pyrrolidine-5'-carboxylic acid, 1'-[N-(1-R-carbethoxy-3-phenylpropyl)-S-alanyl]-(3'R,5'R)-spirobicyclo[2.2.2]octane-2,3'-pyrrolidine-5'-carboxylic acid, 1'-[N-(1-S-carbethoxy-3-phenylpropyl)-R-alanyl]-(3'S,5'S)-spirobicyclo[2.2.2]octane-2,3'-pyrrolidine-5'-carboxylic acid, 1'-[N-(1-S-carbethoxy-3-phenylpropyl)-R-alanyl]-(3'R,5'S)-spirobicyclo[2.2.2]octane-2,3'-pyrrolidine-5'-carboxylic acid, 1'-[N-(1-S-carbethoxy-3-phenylpropyl)-R-alanyl]-(3,S,5'R)-spirobicyclo-[2.2.2]octane-2,3'-pyrrolidine-5'-carboxylic acid, 1'-[N-(1-S-carbethoxy-3-phenylpropyl)-R-alanyl]-(3'R,5'R)-spirobicyclo[2.2.2]octane-2,3'-pyrrolidine-5'-carboxylic acid, 1'-[N-(1-R-carbethoxy-3-phenylpropyl)-R-alanyl]-(3'S,5'S)-spirobicyclo[2.2.2]octane-2,3'-pyrrolidine-5'-carboxylic acid, 1'-[N-(1-R-carbethoxy-3-phenylpropyl)-R-alanyl]-(3'R,5'S)-spirobicyclo[2.2.2]octane-2,3'-pyrrolidine-5'carboxylic acid, 1'-[N-(1-R-carbethoxy-3-phenylpropyl)-R-alanyl]-(3'S,5'R)-spirobicyclo[2.2.2]octane-2,3'-pyrrolidine-5'-carboxylic acid or 1'-[N-(1-R-carbethoxy-3-phenylpropyl)-R-alanyl]-(3'R,5'R)-spirobicyclo[2.2.2]octane-2,3'-pyrrolidine-5'-carboxylic acid.

## Revendications

1. Utilisation d'inhibiteurs d'ACE de formule (II) ou de leurs sels physiologiquement acceptables dans la fabrication d'un médicament à action nootrope, dans la formule (II)

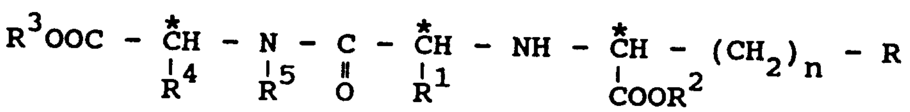

(II)

n étant 1 ou 2,

R représentant un atome d'hydrogène ou un radical aliphatique ayant de 1 à 21 atomes de carbone, ou un radical aromatique ayant de 6 à 12 atomes de carbone,

$R^1$ représentant un atome d'hydrogène ou un radical aliphatique ayant de 1 à 21 atomes de carbone, ou, si elle n'est pas déjà englobée par les définitions précédentes, la chaîne latérale, protégée si nécessaire, d'un α-aminoacide existant dans la nature,

$R^2$ et $R^3$ étant identiques ou différents et représentant un atome d'hydrogène, un radical aliphatique ayant de 1 à 21 atomes de carbone, un radical alicyclique ayant de 3 à 20 atomes de carbone, un radical aromatique ayant de 6 à 12 atomes de carbone, un radical araliphatique ayant de 7 à 32 atomes de carbone, et

$R^4$ et $R^5$ formant ensemble, avec les atomes qui les portent, un système cyclique mono-, bi- ou tricyclique, choisi parmi la pyrrolidine, la tétrahydroisoquinoléine, la décahydroisoquinoléine, l'octahydroindole, l'indoline, l'octahydrocyclopenta[b]pyrrole, le 2-azaspiro[4.5]-décane, le 2-azaspiro[4.4]nonane, la spiro[(bicyclo[2.2.1]heptane)-2,3'-pyrrolidine], la spiro[(bicyclo[2.2.2]octane)-2,3'-pyrrolidine], le 2-azatricyclo[$4.3.0.1^{6,9}$]décane, le décahydrocyclohepta[b]pyrrole, l'octahydroisoindole, l'octahydrocyclopenta[c]pyrrole, le 2,3,3a,4,5,6a-hexahydroindole, le 1,2,3,3a,4,5a-hexahydrocyclopenta[b]pyrroleet le 2-

azabicyclo[3.1.0]hexane.

**2.** Utilisation selon la revendication 1, caractérisée en ce que l'on utilise l'acide (S,S,S,S,S)-1-[N-(1-carbéthoxy-3-phénylpropyl)-alanyl]-octahydroindol-2-carboxylique, l'acide 1-[N-(1-S-carbéthoxy-3-phénylpropyl)-S-alanyl]-(2S,3aR,7aS)-octahydroindol-2-carboxylique, l'acide (S,S,S,S,S)-2-[N-(1-carbéthoxy-3-phénylpropyl)-alanyl]-décahydroisoquinoléine-3-carboxylique, l'acide (S,S,S)-2-[N-(1-carbéthoxy-3-phénylpropyl)-alanyl]-tétrahydroisoquinoléine-3-carboxylique, l'acide (S,S,S,S,S)-2-[N-(1-carbéthoxy-3-phénylpropyl)-alanyl]-2-azabicyclo[3.3.0]octane-3-carboxylique, l'acide 2-N-(1-S-carbéthoxyl-3-phénylpropyl)-S-alanyl]-*cis*,endo-2-azabicyclo[3.1.0]hexane-3-S-carboxylique ou l'acide 1-[N-(1-S-carbéthoxy-3-phénylpropyl)-S-alanyl]-*cis*,endo-1H-2,3,3a,4,5,7a-hexahydroindol-2-S- ou -2-R-carboxylique.

**3.** Utilisation selon la revendication 1, caractérisée en ce que l'on utilise l'acide 1'-[N-(1-S-carbéthoxy-3-phénylpropyl)-S-alanyl]-exo/endo-spirobicyclo[2.2.2]octane-2,3-pyrrolidine-5'-carboxylique, l'acide 1'-[N-(1-S-carbéthoxy-3-phénylpropyl)-S-alanyl]-(3'S,5'S)-spirobicyclo[2.2.2]octane-2,3'-pyrrolidine-5'-carboxylique, l'acide 1'-[N-(1-S-carbéthoxy-3-phénylpropyl)-S-alanyl]-(3'S,5'R)-spirobicyclo[2.2.2]-octane-2,3'-pyrrolidine-5'-carboxylique, l'acide 1'-[N-(1-S-carbéthoxy-3-phénylpropyl)-S-alanyl]-(3'R,5'S)-spirobicyclo[2.2.2]octane-2,3'-pyrrolidine-5'-carboxylique, l'acide 1'-[N-(1-S-carbéthoxy-3-phénylpropyl)-S-alanyl]-(3'R,5'R)-spirobicyclo[2.2.2]octane-2,3'-pyrrolidine-5'-carboxylique, l'acide 1'-[N-(1-R-carbéthoxy-3-phénylpropyl)-S-alanyl]-(3'S,5'S)-spirobicyclo[2.2.2]octane-2,3'-pyrrolidine-5'-carboxylique, l'acide 1'-[N-(1-R-carbéthoxy-3-phénylpropyl)-S-alanyl]-(3'S,5'R)-spirobicyclo[2.2.2]-octane-2,3'-pyrrolidine-5'-carboxylique, l'acide 1'-[N-(1-R-carbéthoxy-3-phénylpropyl)-S-alanyl]-(3'R,5'S)-spirobicyclo[2.2.2]octane-2,3'-pyrrolidine-5'-carboxylique, l'acide 1'-[N-(1-R-carbéthoxy-3-phénylpropyl)-S-alanyl]-(3'R,5'R)-spirobicyclo[2.2.2] octane-2,3'-pyrrolidine-5'-carboxylique, l'acide 1'-[N-(1-S-carbéthoxy-3-phénylpropyl)-R-alanyl]-(3'S,5'S)-spirobicyclo[2.2.2]octane-2,3'-pyrrolidine-5'-carboxylique, l'acide 1'-[N-(1-S-carbéthoxy-3-phénylpropyl)-R-alanyl]-(3'R,5'S)-spirobicyclo[2.2.2]-octane-2,3'-pyrrolidine-5'-carboxylique, l'acide 1'-[N-(1-S-carbéthoxy-3-phénylpropyl)-R-alanyl]-(3'S, 5'R)-spirobicyclo[2.2.2]octane-2,3'-pyrrolidine-5'-carboxylique, l'acide 1'-[N-(1-S-carbéthoxy-3-phénylpropyl)-R-alanyl]-(3'R,5'R)-spirobicyclo[2.2.2]octane-2,3'-pyrrolidine-5'-carboxylique, l'acide 1'-[N-(1-R-carbéthoxy-3-phénylpropyl)-R-alanyl]-(3'S,5'S)-spirobicyclo[2.2.2]octane-2,3'-pyrrolidine-5'-carboxylique, l'acide 1'-[N-(1-R-carbéthoxy-3-phénylpropyl)-R-alanyl]-(3'R,5'S)-spirobicyclo[2.2.2]octane-2,3'-pyrrolidine-5'-carboxylique, l'acide 1'-[N-(1-R-carbéthoxy-3-phénylpropyl)-R-alanyl]-(3'S,5'R)-spirobicyclo[2.2.2]-octane-2,3'-pyrrolidine-5'-carboxylique ou l'acide 1'-[N-(1-R-carbéthoxy-3-phénylpropyl)-R-alanyl]-(3'R,5'R)-spirobicyclo[2.2.2]octane-2,3'-pyrrolidine-5'-carboxylique.